(19) Europäisches Patentamt
European Patent Office
Office européen des brevets

(11) Numéro de publication : **0 434 560 B1**

(12) **FASCICULE DE BREVET EUROPEEN**

(45) Date de publication du fascicule du brevet : **26.01.94 Bulletin 94/04**

(51) Int. Cl.$^5$ : **C07K 5/06,** A61K 37/64

(21) Numéro de dépôt : **90403687.8**

(22) Date de dépôt : **20.12.90**

(54) **Nouveaux dérivés d'amino-acides substitués, leur procédé de préparation et les compositions pharmaceutiques qui les contiennent.**

(30) Priorité : **20.12.89 FR 8916881**

(43) Date de publication de la demande : **26.06.91 Bulletin 91/26**

(45) Mention de la délivrance du brevet : **26.01.94 Bulletin 94/04**

(84) Etats contractants désignés : **AT BE CH DE DK ES FR GB GR IT LI LU NL SE**

(56) Documents cités :
**EP-A- 0 105 102**
**EP-A- 0 113 880**
**EP-A- 0 305 297**

(73) Titulaire : **ADIR ET COMPAGNIE**
**1 rue Carle Hébert**
**F-92415 Courbevoie Cédex (FR)**

(72) Inventeur : **Vincent, Michel**
**8 allée du Prunier Hardy**
**92220 Bagneux (FR)**
Inventeur : **Remond, Georges**
**9 avenue des Etats-Unis**
**78000 Versailles (FR)**
Inventeur : **Portevin, Bernard**
**6 rue Frédéric Passy**
**78990 Elancourt (FR)**
Inventeur : **Herve, Yolande**
**16 rue Eichenberger**
**92800 Puteaux (FR)**
Inventeur : **Lepagnol, Jean**
**5 rue de Vlaminck**
**78400 Chatou (FR)**

**EP 0 434 560 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés d'amino-acides substitués, leur procédé de préparation, les compositions pharmaceutiques qui les contiennent.

Parmi les inhibiteurs de l'enzyme de conversion connus, quelques uns d'entre eux ont également été décrits comme agents nootropes. C'est le cas de composés mentionnés dans les demandes de brevets européens EP 307 872 et EP 288 907 et EP 243 645 qui présentent des propriétés en tant qu'inhibiteurs des pertes de fonctions cognitives.

Les composés de la présente invention, outre le fait qu'ils soient nouveaux, se différencient des nootropes de l'art antérieur par l'intensité de leurs effets pharmacologiques.

En effet, ils exercent une activité facilitatrice des fonctions mnésiques à des doses minima bien plus faibles que celles du nootrope le plus actif pris en référence, l'oxiracétam.

Cet effet promnésique s'exerce notamment par le biais de la neurotransmission cholinergique. De plus, les dérivés de l'invention possèdent un effet inhibiteur de l'enzyme de conversion de l'angiotensine I en angiotensine II, ce qui leur confère un effet antihypertenseur et un effet protecteur vis à vis des conséquences de l'hypertension, l'un des facteurs de risque les plus importants en pathologie cérébrale (accident vasculaire cérébral, démence multi-infarctus).

Ainsi, ces différentes propriétés impliquent que les dérivés de l'invention semblent beaucoup plus aptes à une utilisation en thérapeutique que ceux de structure proche décrits dans la littérature.

L'invention concerne plus particulièrement de nouveaux dérivés d'amino-acides bicycliques répondant à la formule générale (I) :

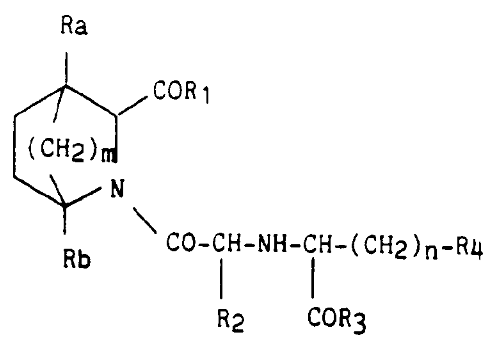

(I)

dans laquelle :

- $R_1$ représente un groupement alcoxy inférieur ou supérieur linéaire ou ramifié, ou un groupement amino éventuellement substitué par un ou deux groupements alkyle inférieur linéaire ou ramifié,
- $R_2$ représente un groupement alkyle inférieur linéaire ou ramifié éventuellement substitué par un groupement amino,
- $R_3$ représente un groupement amino, un groupement alcoxy inférieur ou supérieur linéaire ou ramifié, un groupement hydroxyle, à condition toutefois qu'au moins un groupement amino soit présent en $R_1$ ou $R_3$,
- $R_4$ représente un atome d'hydrogène ou un groupement aryle,
- m est égal à 1 ou 2,
- n est compris entre 1 et 6,
- Ra et Rb identiques ou différents représentent quand m = 1, un atome d'hydrogène, quand m = 2, un groupement alkyle inférieur linéaire ou ramifié ou un atome d'hydrogène,

  le terme inférieur indiquant que les groupements ainsi qualifiés comptent de 1 à 6 atomes de carbone,

  le terme supérieur indiquant que les groupements ainsi qualifiés comptent de 7 à 12 atomes de carbone,

leurs énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, sulfurique, tartrique, maléique, fumarique, oxalique, méthane sulfonique, camphorique, etc...

Parmi les bases pharmaceutiquement acceptables, on peut citer à titre non limitatif l'hydroxyde de sodium, l'hydroxyde de potassium, la tertbutylamine, etc...

L'invention s'étend aussi au procédé de préparation des dérivés de formule (I) caractérisé en ce que l'on soumet à une amination réductrice le composé de formule (II) :

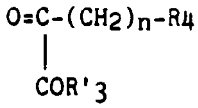

(II)

dans laquelle $R_4$ a la même signification que dans la formule (I), et $R'_3$ représente un groupement amino ou alcoxy inférieur ou supérieur, linéaire ou ramifié, en présence d'un hydrure mixte de métal alcalin tel que le cyanoborohydrure de sodium avec un amino-acide de formule (III) dont la fonction acide est protégée :

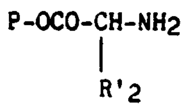

(III)

dans laquelle P est un groupement alkyle tel que le terbutyle et $R'_2$ un groupement alkyle inférieur linéaire ou ramifié éventuellement substitué par un groupement amino lui-même protégé par un goupement protecteur tel que le benzyloxycarbonyle,
pour obtenir un dérivé de formule (IV) :

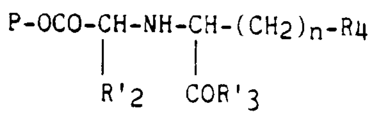

(IV)

dans laquelle $R'_2$, $R'_3$, $R_4$, P et n ont les mêmes significations que précédemment, dont on sépare éventuellement les isomères par une technique classique de séparation et que l'on déprotège en milieu acide pour obtenir un dérivé de formule (V) :

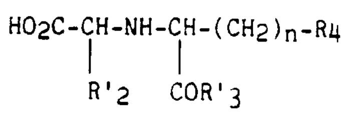

(V)

dans laquelle $R'_2$, $R'_3$, $R_4$ et n ont les mêmes significations que précédemment,
qui est alors couplé avec un dérivé de formule (VI) selon la technique de couplage peptidique décrite par W. KONIG et R. GEIGER (Ber. 103, 788, 1970) :

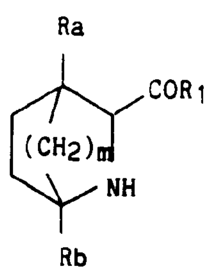

(VI)

dans laquelle Ra, Rb, m et $R_1$ ont les mêmes significations que dans la formule (I),
pour conduire à un dérivé de formule (I/a) cas particulier des dérivés de formule (I) :

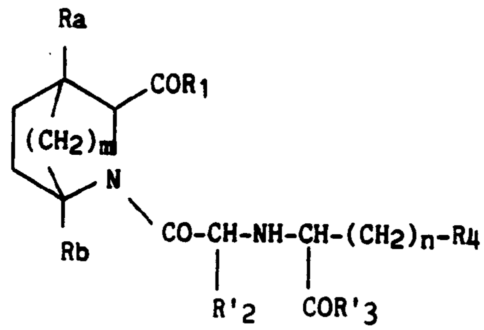

(I/a)

dans laquelle Ra, Rb, $R_1$, $R'_2$, $R'_3$, $R_4$, m et n ont la même signification que précédemment, qui, si on le désire, lorsque $R'_2$ représente un groupement alkyle inférieur linéaire ou ramifié et $R'_3$ est un groupement alcoxy inférieur ou supérieur linéaire ou ramifié peut être soumis à l'action d'une base ou d'un acide pour conduire à un dérivé de formule (I/b), cas particulier des dérivés de formule (I) :

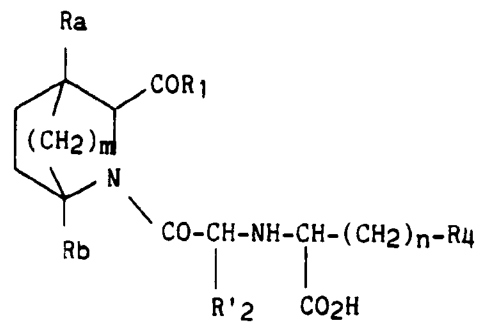

(I/b)

dans laquelle Ra, Rb, $R_1$, $R_4$, m, n ont la même signification que dans la formule (I), $R'_2$ représente un groupement alkyle inférieur linéaire ou ramifié et $R_3$ représente un groupement hydroxyle, ou bien, lorsque $R'_2$ est un groupement alkyle substitué par un groupement amino protégé et $R'_3$ un groupement alcoxy inférieur ou supérieur, linéaire ou ramifié, soumis à une hydrogénation en présence d'un catalyseur tel que le charbon palladié, pour conduire à un dérivé de formule (I/c), cas particulier des dérivés de formule (I) :

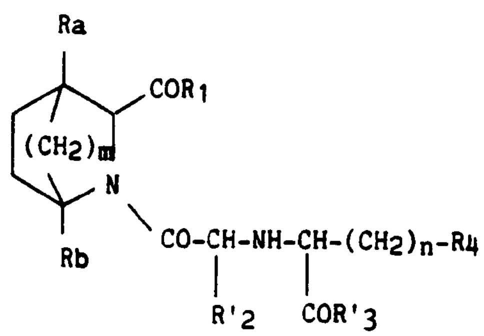

(I/c)

dans laquelle Ra, Rb, $R_1$, $R_4$, n et m ont les mêmes significations que dans la formule (I), $R'_2$ représente un groupement alkyle substitué par un groupement amino et $R'_3$ représente un groupement alcoxy inférieur ou supérieur linéaire ou ramifié,
dérivé (I/c) qui, si on le désire, peut être soumis à l'action d'une base, pour conduire à un dérivé de formule (I/d), cas particulier des dérivés de formule (I) :

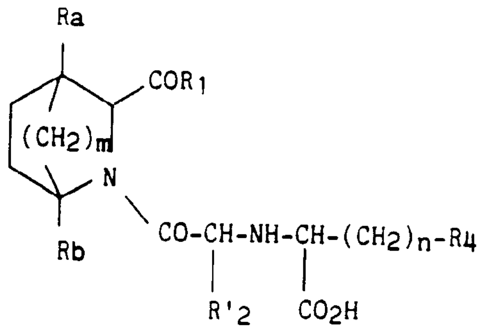

(I/d)

dans laquelle Ra, Rb, $R_1$, $R_4$, n et m ont les mêmes significations que dans la formule (I), $R'_2$ représente un groupement alkyle substitué par un groupement amino et $R_3$ représente un groupement hydroxyle,
dérivés de formule (I/a), (I,b), (I/c) et (I/d) que l'on purifie par une technique classique de purification, dont on sépare éventuellement les isomères par une technique classique de séparation et que l'on transforme, si on le désire, en leurs sels d'addition à un acide ou à une base pharmaceutiquement acceptable.

Les composés de formule (VI) dans laquelle $R_1$ représente un groupement alcoxy supérieur sont nouveaux et font partie de l'invention au même titre que les composés de formule (I) dont ils constituent les intermédiaires de synthèse.

Les composés de formule (I) possèdent des propriétés pharmacologiques très intéressantes.

Ils sont d'une part doués d'effets inhibiteurs de l'enzyme de conversion de l'angiotensine I et d'autre part, d'effets antagonistes vis à vis de l'amnésie à la scopolamine. Par leur première propriété, ils peuvent donc s'opposer à la maladie hypertensive artérielle et à ses conséquences notamment cérébrales et conférer aux patients une sensation de bien-être comme il a été décrit précédemment pour le premier IEC connu, le Captopril.

Par la seconde propriété, ils facilitent les performances d'apprentissage et de remémoration, dont le dysfonctionnement est largement démontré lors du vieillissement et plus encore lors des démences dégénératives préséniles, séniles et vasculaires.

Cette facilitation est remarquablement observée lors d'amnésie expérimentale induite par un antagoniste cholinergique.

Les effets promnésiques des dérivés de la présente invention concernent donc en particulier la neurotransmission cholinergique dont l'implication étroite dans les fonctions de mémoire est unanimement reconnue.

L'ensemble de ces propriétés confère donc aux présents dérivés des propriétés protectrices cardiovasculaires et cérébrales et des propriétés facilitatrices des fonctions mnésiques qui sont toujours la résultante des capacités d'attention, de vigilance, de bien-être et de mémorisation.

Les effets mnésiques de ces composés s'exercent de façon beaucoup plus intense que ceux du plus récent nootrope qui a été pris en référence : l'oxiracétam.

Ces composés s'avèrent donc utiles pour le traitement thérapeutique, préventif ou curatif, des troubles neurocomportementaux associés à l'accident vasculaire cérébral, au vieillissement et aux démences dégénératives séniles ou préséniles telles que la maladie d'Alzheimer, la maladie de Pick, la démence par multi-infarctus et la maladie de Binswanger.

La présente invention a également pour objet les compositions pharmaceutiques renfermant comme principe actif au moins un composé de formule générale (I) ou un de ses sels d'addition à un acide ou à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 100 mg pour un traitement en 1 à 3 prises par 24 heures.

Les exemples suivants illustrent l'invention et ne la limitent en aucune façon.

**EXEMPLE 1 : [[(ETHOXYCARBONYL)-1 BUTYLAMINO-(S)]-2 PROPIONYL-(S)]-2 CARBAMOYL-3(S) AZA-2 BICYCLO [2.2.2] OCTANE**

En utilisant la méthode de couplage peptidique (DCC/HOBT) décrite par W. KONIG et R. GEIGER (Ber, 103, 788, 1970), on prépare, à partir de 0,02 mole de carbamoyl-(S)-3 aza-2 bicyclo [2.2.2] octane décrit dans le brevet FR 2585709 et de 0,02 mole de N-[(éthoxycarbonyl)-1 butyl-(S)]-(S) alanine décrite par VINCENT et al (Tetrahedron Letters 23, (1982), 1677-1680), le produit attendu qui est purifié par chromatographie sur gel de silice (solvant d'élution : dichlorométhane/éthanol : 90/10) puis lyophilisé.

Rendement : 58%

Microanalyse élémentaire :

| | C% | H% | N% |
|---|---|---|---|
| calculé : | 61,17 | 8,84 | 11,89 |
| trouvé : | 61,01 | 9,04 | 11,93 |

Résonance magnétique nucléaire du proton ($CDCl_3$) :

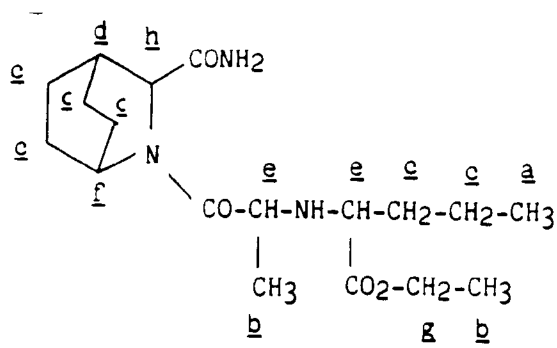

a δ = 0,9 ppm (3H,m)
b δ = 1,3 ppm (6H,m)
c δ = 1,6 ppm (12H,m)
d δ = 2,0 ppm (1H,m)
e δ entre 3,1 et 3,6 ppm (2H,m)
f δ = 4,0 ppm (1H,m)
g δ = 4,2 ppm (2H,q)
h δ = 4,4 ppm (1H,m)

**EXEMPLE 2 : [[CARBOXY-1BUTYLAMINO-(S)]-2 PROPIONYL-(S)]-2 CARBAMOYL-3-(S) AZA-2 BICYCLO [2.2.2] OCTANE**

4,25 mmoles (1,5 g) du composé obtenu dans l'exemple 1 sont dissous dans 50 cm³ de soude 0,1N. La solution est abandonnée 24 heures à température ambiante, neutralisée par addition d'acide chlorhydrique 1N puis évaporée à sec. Le résidu est repris par 50 cm³ d'isopropanol, filtré et évaporé puis repris dans 40 cm³ d'eau. Le produit attendu est obtenu après lyophilisation.

Rendement : 94%

Résonance magnétique nucléaire du proton (DMSO d6) :

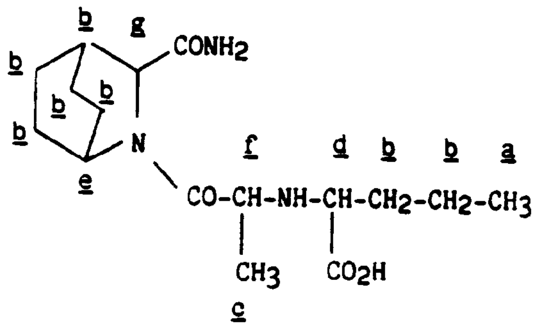

a δ = 0,9 ppm (3H,t)
b δ = entre 1,0 et 2,4 ppm (13H,m)
c δ = 1,2 ppm (3H,d)
d δ = 3,05 ppm (1H,t)
e δ = 3,85 ppm (1H,m)
f δ = 3,9 ppm (1H,q)
g δ = 4,1 ppm (1H,d)

**EXEMPLE 3 : [[CARBAMOYL-1 BUTYLAMINO-(S)]-2 PROPIONYL-(S)]-2 CARBAMOYL-3-(S) AZA-2 BICYCLO [2.2.2] OCTANE**

**STADE A : N-[CARBAMOYL-1 BUTYL-(S)]-(S) ALANINE**

10 g de N-[éthoxycarbonyl-1 butyl-(S)]-(S) alanine sont placés dans 100 ml d'une solution aqueuse ammoniacale à 28% à 100°C pendant 18 heures. Après évaporation du solvant le résidu est repris par 50 ml d'eau. La solution est évaporée à sec puis reprise par 100 ml d'isopropanol et évaporée. Le produit attendu est obtenu après séchage sous vide.

Rendement : 95%

Résonance magnétique nucléaire du proton (DMSO d6) :

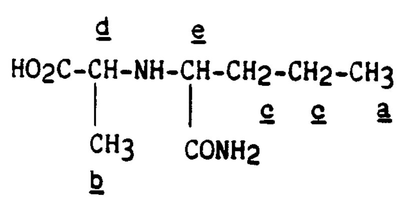

a δ = 0,9 ppm (3H,t)
b δ = 1,3 ppm (3H,d)
c δ entre 1,2 et 1,7 ppm (4H,m)
d δ = 3,1 ppm (1H,q)
e δ = 3,2 ppm (1H,m)

**STADE B : [[CARBAMOYL-1 BUTYLAMINO-(S)]-2 PROPIONYL-(S)]-2 CARBAMOYL-3-(S) AZA-2 BICYCLO [2.2.2] OCTANE**

En procédant comme dans l'exemple 1, mais en remplaçant la N-[ (éthoxycarbonyl)-1 butyl-(S)]-(S) alanine par la N-[carbamoyl-1 butyl-(S)]-(S) alanine obtenue au stade A, on obtient le produit attendu qui est purifié

par chromatographie sur gel de silice (solvant d'élution : dichlorométhane / méthanol / ammoniaque : 90/10/0,5)

Rendement : 22%

Microanalyse élémentaire :

| | C% | H% | N% |
|---|---|---|---|
| calculé : | 59,24 | 8,70 | 17,27 |
| trouvé : | 59,38 | 8,78 | 17,35 |

Résonance magnétique nucléaire du proton (DMSO $d_6$) :

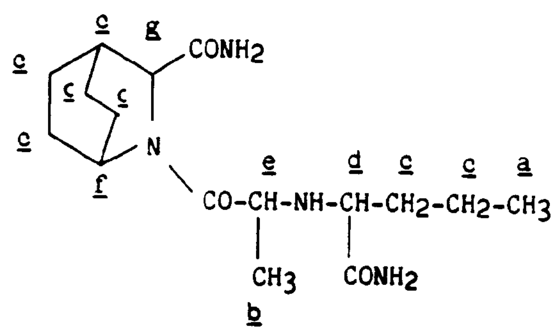

a δ = 0,8 ppm (3H,t)
b δ = 1,1 ppm (3H,d)
c δ entre 1,2 et 2,3 ppm (13H,m)
d δ = 2,7 ppm (1H,m)
e δ = 3,4 ppm (1H,m)
f δ = 3,8 ppm (1H,m)
g δ = 4,1 ppm (1H,m)

**EXEMPLE 4 : ESTER n-OCTYLIQUE DE L'ACIDE [[CARBAMOYL-1 BUTYLAMINO-(S)]-2 PROPIONYL-(S)]-2 AZA-2 BICYCLO [2.2.2] OCTANECARBOXYLIQUE-3-(S)**

**STADE A : CHLORYDRATE DE L'ESTER n-OCTYLIQUE DE L'ACIDE AZA-2 BICYCLO [2.2.2] OCTANECARBOXYLIQUE-3-(S)**

Dans un ballon de 250 ml contenant 60 ml de n-octanol refroidi à 0°C, 9,6 ml de chlorure de thionyle sont additionnés goutte à goutte. après 10 mn d'agitation à 0°C, 18,6 g d'acide aza-2 bicyclo [2.2.2] octanecarboxylique-3-(S) (décrit dans le brevet Fr 2585709) sont ajoutés lentement. Le mélange réactionnel est laissé sous agitation une nuit à température ambiante puis chauffé 6 heures à 80°C et à nouveau abandonné une nuit à température ambiante. Après addition de 100 ml d'éther anhydre et filtration du chlorhydrate n'ayant pas réagi, le filtrat est condensé puis repris par un mélange eau / éther (50/50). Le produit attendu est obtenu après concentration de la phase aqueuse puis séchage sous vide.

Rendement : 81%

Microanalyse élémentaire :

| | C% | H% | N% | Cl |
|---|---|---|---|---|
| calculé : | 63,24 | 9,95 | 4,61 | 11,67 |
| trouvé : | 62,90 | 9,93 | 4,65 | 11,33 |

**STADE B : ESTER n-OCTYLIQUE DE L'ACIDE [[CARBAMOYL-1 BUTYLAMINO-(S)]-2 PROPIONYL-(S)]-2 AZA-2 BICYCLO [2.2.2] OCTANECARBOXYLIQUE-3-(S)**

En procédant comme dans l'exemple 1, mais en remplaçant la N-[(éthoxycarbonyl)-1 butyl-(S)]-(S) alanine par la N-[carbamoyl-1 butyl-(S)]-(S) alanine décrite au stade A de l'exemple 3 et le carbamoyl-(S)-3 aza-2 bicyclo-[2.2.2] octane par le chlorhydrate de l'ester n-octylique de l'acide aza-2 bicyclo [2.2.2] octane carboxylique-3-(S) obtenu au stade A (neutralisé préalablement par de la triéthylamine), on obtient le produit attendu qui est purifié par chromatographie sur gel de silice (solvant d'élution : dichlorométhane / éthanol : 95/5).

Rendement : 21%

Résonance magnétique nucléaire du proton ($CDCl_3$) :

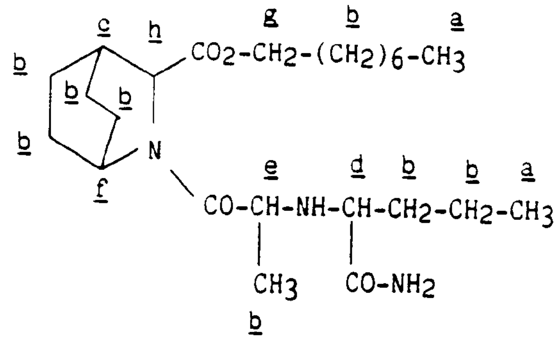

a δ = 0,9 ppm (6H,m)
b δ entre 1,15 et 1,90 ppm (27H,m)
c δ = 2,05 ppm (1H,m)
d δ = 2,85 ppm (1H,t)
e δ = 3,4 ppm (1H,q)
f δ = 3,8 ppm (1H,m)
g δ entre 4,00 et 4,25 ppm (2H,m)
h δ = 4,4 ppm (1H,m)

**EXEMPLE 5 : [[(ETHOXYCARBONYL)-1 PHENYL-3 PROPYLAMINO -(S)]-2 PROPIONYL-(S)]-2 CARBAMOYL-3-(S) AZA-2 BICYCLO [2.2.2] OCTANE**

En procédant comme dans l'exemple 1, mais en remplaçant la N-[(éthoxycarbonyl)-1 butyl-(S)]-(S) alanine par la N-[(éthoxycarbonyl)-1 phenyl-3 propyl]-(S) alanine décrite par J.S. KALTENBROWN et al. (Organic Preparations Procedures Int. 15 (1-2), 35-40 (1983)), on obtient le produit attendu qui est purifié, après dissolution dans de l'acide chlorhydrique 0,5N, filtration de l'insoluble, neutralisation du filtrat puis filtration du précipité et lavage à l'eau.

Rendement : 85%

Résonance magnétique nucléaire du proton (DMSO $d_6$) :

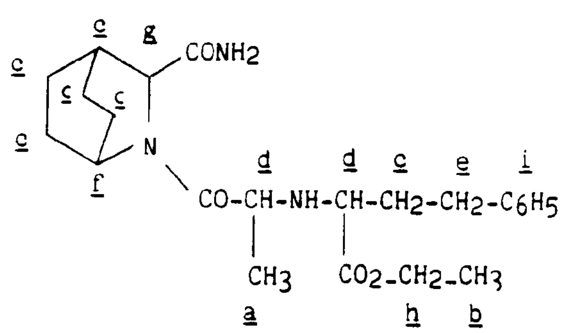

a δ = 1,1 ppm (3H,d)
b δ = 1,2 ppm (3H,t)
c δ entre 1,3 et 2,3 ppm (11H,m)
d δ = 2,6 ppm (2H,m)
e δ = 3,2 ppm (2H,m)
f δ = 3,5 ppm (1H,m)
g δ = 4,0 ppm (1H,m)
h δ = 4,1 ppm (2H,q)
i δ = 7,2 ppm (5H,m)

**EXEMPLE 6 : [[(CARBOXY-1 PHENYL-3 PROPYLAMINO -(S)]-2 PROPIONYL-(S)]-2 CARBAMOYL-3-(S) AZA-2 BICYCLO [2.2.2] OCTANE**

1 g du composé obtenu dans l'exemple 5 est mis en solution dans 50 cm³ d'acide chlorhydrique 6N puis porté 8 heures à reflux. Après évaporation, le résidu est dissous dans 50 cm³ d'eau puis fixé sur résine DOWEX 50 WX8. Après lavage à l'eau, le produit est élué par un mélange eau / pyridine (90/10). Le produit attendu est obtenu après évaporation, purifié par chromatographie sur gel de silice (solvant d'élution acétone / eau : 90/10), repris à l'eau puis lyophilisé.

Rendement : 13%

Résonance magnétique nucléaire du proton (DMSO $d_6$) :

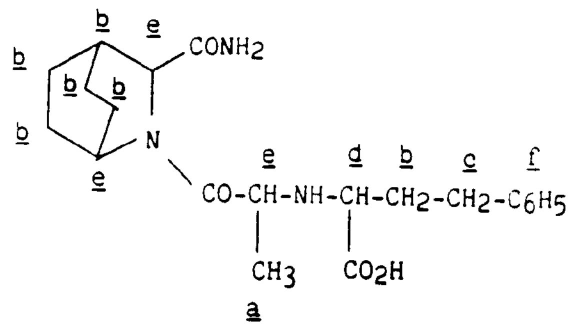

a δ = 1,3 ppm (3H,d)
b δ entre 1,2 et 2,2 ppm (11H,m)
c δ = 2,6 ppm (2H,m)
d δ = 3,1 ppm (1H,t)
e δ = 4,0 ppm (3H,m)
f δ = 7,2 ppm (5H,m)

**EXEMPLE 7 : [[(ETHOXYCARBONYL)-1 PHENYL-3 PROPYLAMINO -(S)]-2 AMINO-6 HEXANOYL-(S)]-2 CARBAMOYL-3-(S) AZA-2 BICYCLO [2.2.2] OCTANE**

**STADE A : [[(ETHOXYCARBONYL)-1 PHENYL-3 PROPYLAMINO -(S)]-2 BENZYLOXYCARBONYLAMINO-6 HEXANOYL-(S)]-2 CARBAMOYL-3-(S) AZA-2 BICYCLO [2.2.2] OCTANE**

En procédant comme dans l'exemple 1, mais en remplaçant la N-[(éthoxycarbonyl)-1 butyl(S)]-(S) alanine par l'acide [(éthoxycarbonyl)-1 phényl-3 propylamino-(S)]-2 benzyloxycarbonylamino-6 hexanoïque (S) décrit dans le brevet Fr 2619813, on obtient le produit attendu.

Rendement : 63%

Microanalyse élémentaire :

| | C% | H% | N% |
|---|---|---|---|
| calculé : | 67,30 | 7,64 | 9,23 |
| trouvé : | 67,07 | 7,64 | 9,26 |

**STADE B : [[(ETHOXYCARBONYL)-1 PHENYL-3 PROPYLAMINO -(S)]-2 AMINO-6 HEXANOYL-(S)]-2 CARBAMOYL-3-(S) AZA-2 BICYCLO [2.2.2] OCTANE**

6,4 g du produit obtenu au stade A sont dissous dans 60 ml d'éthanol anhydre. Après addition de 0,5 g de charbon palladié à 10% et hydrogénolyse à température ambiante pendant 20 heures sous une pression d'hydrogène de 3 kg/cm$^2$, le mélange est filtré et évaporé. Le résidu est repris par 100 ml d'eau. La solution est filtrée et le produit attendu est obtenu après lyophilisation.

Rendement : 82%

Résonance magnétique nucléaire du proton ($CDCl_3$) :

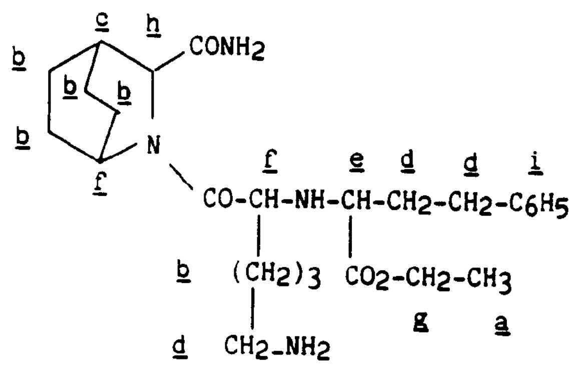

a δ = 1,3 ppm (3H,t)
b δ = 1,7 ppm (14H,m)
c δ = 2,2 ppm (1H,m)
d δ = 2,7 ppm (6H,t)
e δ = 3,1 ppm (1H,m)
f δ entre 3,5 et 4,1 ppm (2H,m)
g δ = 4,2 ppm (2H,q)
h δ = 4,4 ppm (1H,m)
i δ = 7,3 ppm (5H,m)

**EXEMPLE 8 : [[CARBOXY-1 PHENYL-3 PROPYLAMINO-(S)]-2 AMINO-6 HEXANOYL-(S)]-2 CARBAMOYL-3-(S) AZA-2 BICYCLO [2.2.2] OCTANE**

En procédant comme dans l'exemple 2, mais en remplaçant le composé obtenu dans l'exemple 1 par l'[[(éthoxycarbonyl)-1 phényl-3 propylamino-(S)]-2 amino-6 hexanoyl-(S)]-2 carbamoyl-3-(S) aza-2 bicyclo [2.2.2] octane obtenu dans l'exemple 7, on obtient le produit attendu.

Rendement : 72%

Résonance magnétique nucléaire du proton (DMSO d6) :

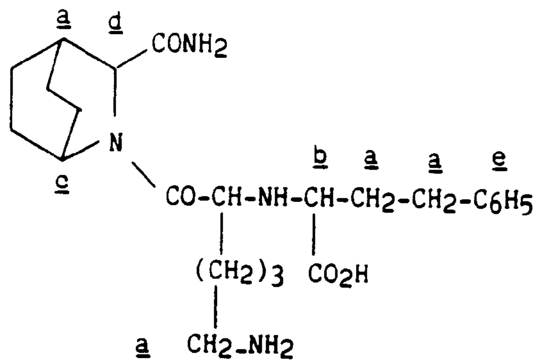

a δ entre 2,1 et 3,0 ppm (7H,m)
b δ = 3,4 ppm (1H,m)
c δ = 3,8 ppm (1H,m)
d δ = 4,05 ppm (1H,m)
e δ = 7,1 ppm (5H,m)

**EXEMPLE 9 : [[CARBAMOYL-1 PHENYL-3 PROPYLAMINO-(S)]-2 PROPIONYL-(S)]-2 CARBAMOYL-3-(S) AZA-2 BICYCLO [2.2.2] OCTANE**

**STADE A : N-[CARBAMOYL-1 PHENYL-3 PROPYL-(S)]-(S) ALANINE**

En procédant comme au stade A de l'exemple 3, mais en remplaçant la N-[(éthoxycarbonyl)-1 butyl-(S)]-(S) alanine par la N-[éthoxycarbonyl-1 phényl-3 propyl-(S)]-(S) alanine, on obtient le produit attendu.

Rendement : 96%

Résonance magnétique nucléaire du proton (DMSO $d_6$) :

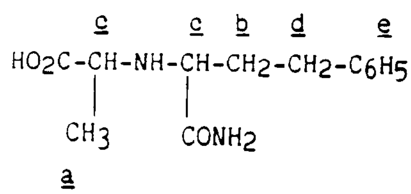

a δ = 1,25 ppm (3H,d)
b δ entre 1,7 et 2,0 ppm (2H,m)
c δ entre 3,0 et 3,35 ppm (2H,m)
d δ = 2,6 ppm (2H,m)
e δ = 7,3 ppm (5H,m)

**STADE B : [[CARBAMOYL-1 PHENYL-3 PROPYLAMINO-(S)]-2 PROPIONYL-(S)]-2 CARBAMOYL-3-(S) AZA-2 BICYCLO [2.2.2] OCTANE**

En procédant comme au stade B de l'exemple 3, mais en remplaçant la N-[carbamoyl-1 butyl-(S)]-(S) alanine par la N-[carbamoyl-1 phényl-3 propyl-(S)]-(S) alanine obtenue au stade précédent, on obtient le produit attendu après purification sur gel de silice (solvant d'élution : dichlorométhane / méthanol / ammoniaque : 90/10/0,5).

Rendement : 24%

Résonance magnétique nucléaire du proton (DMSO $d_6$) :

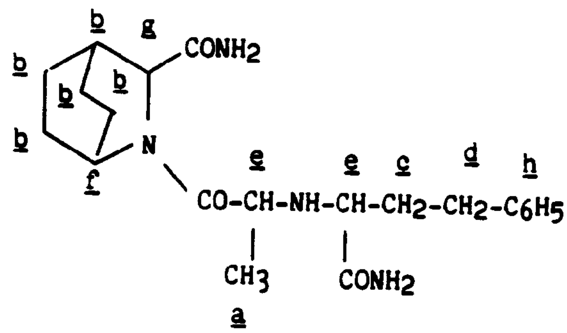

a δ = 1,15 ppm (3H,d)
b δ entre 1,20 et 1,90 ppm (3H,m)
c δ = 1,9 ppm (2H,m)
d δ = 2,65 ppm (2H,m)
e δ = 2,80 ppm (2H,m)
f δ = 3,45 ppm (1H,m)
g δ = 4,15 ppm (1H,m)
h δ = 7,2 ppm (5H,m)

**EXEMPLE 10 : ESTER n-OCTYLIQUE DE L'ACIDE [[CARBAMOYL-1 PHENYL-3 PROPYLAMINO-(S)]-2 AMINO-6 HEXANOYL-(S)]-2 AZA-2 BICYCLO [2.2.2] OCTANE CARBOXYLIQUE-3-(S), DICHLORHYDRATE**

**STADE A : ACIDE [CARBAMOYL-1 PHENYL-3 PROPYLAMINO-(S)]-2 BENZYLOXYCARBONYLAMINO-6 HEXANOÏQUE-(S)**

En procédant comme au stade A de l'exemple 3, mais en remplaçant la N-[éthoxycarbonyl-1 butyl(S)]-(S) alanine par l'acide [éthoxycarbonyl-1 phényl-3 propyl-amino-(S)]-2 benzyloxycarbonylamino-6 hexanoïque-(S), on obtient le produit attendu.

Rendement : 85%

Résonance magnétique nucléaire du proton (DMSO $d_6$) :

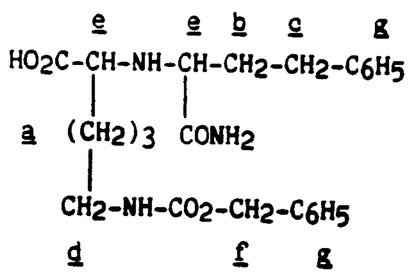

a δ entre 0,7 et 1,8 ppm (6H,m)
b δ = 1,9 ppm (2H,m)
c δ = 2,65 ppm (2H,m)
d δ = 3,0 ppm (2H,m)
e δ entre 3,15 et 3,5 ppm (2H,m)
f δ = 5,00 ppm (2H,s)

g δ = 7,25 ppm (10H,m)

**STADE B : ESTER n-OCTYLIQUE DE L'ACIDE [[CARBAMOYL-1 PHENYL-3 PROPYLAMINO-(S)]-2 BENZYLOXYCARBONYLAMINO-6 HEXANOYL-(S)]-2 AZA-2 BICYCLO [2.2.2] OCTANE CABOXYLIQUE-3-(S)**

En procédant comme au stade B de l'exemple 4, mais en remplaçant la N-[carbamoyl-1 butyl-(S)] alanine-(S) par l'acide [carbamoyl-1 phényl-3 propylamino-(S)]-2 benzyloxycarbonylamino-6 hexanoïque-(S) préparé au stade A, on obtient le produit attendu qui est purifié par chromatographie sur gel de silice (solvant d'élution : dichlorométhane / éthanol : 97/3).

Rendement : 26%

**STADE C : ESTER n-OCTYLIQUE DE L'ACIDE [[CARBAMOYL-1 PHENYL-3 PROPYLAMINO-(S)]-2 AMINO-6 HEXANOYL-(S)]-2 AZA-2 BICYCLO [2.2.2] OCTANE CARBOXYLIQUE-3-(S), DICHLORHYDRATE**

En procédant comme au stade B de l'exemple 7, mais en remplaçant le produit obtenu au stade A de l'exemple 7 par le produit décrit au stade B. Le produit attendu est obtenu après dissolution du résidu huileux dans de l'éther, acidification par de l'éther chlorhydrique, filtration, lavage à l'hexane, dissolution dans l'eau et lyophilisation.

Rendement : 61%

Microanalyse élémentaire :

| | C% | H% | N% | Cl% |
|---|---|---|---|---|
| calculé : | 61,04 | 8,64 | 8,90 | 11,26 |
| trouvé : | 60,66 | 8,54 | 8,75 | 11,16 |

Résonance magnétique nucléaire du proton ($D_2O$) :

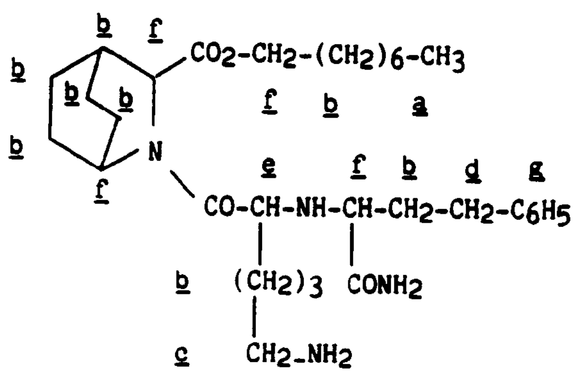

, 2 HCl

a δ = 0,8 ppm (3H,t)
b δ entre 1,0 et 2,1 ppm (29H,m)
c δ = 2,6 ppm (2H,t)
d δ = 2,9 ppm (2H,t)
e δ = 3,6 ppm (1H,t)
f δ vers 4,1 ppm (5H,m)
g δ = 7,1 ppm (5H,m)

**EXEMPLE 11 : [[ETHOXYCARBONYL-1 PHENYL-3 PROPYLAMINO-(S)]-2 PROPIONYL-(S)]-2 CARBAMOYL-3 AZA-2 BICYCLO [2.2.1] HEPTANE, ISOMERE α**

En procédant comme dans l'exemple 5, mais en remplaçant le carbamoyl-(S)-3 aza-2 bicyclo [2.2.2] octane par le carbamoyl-3 aza-2 bicyclo [2.2.1] heptane, isomère a décrit dans le brevet français n° 89.08672 on obtient le produit attendu après chromatographie sur gel de silice (solvant d'élution : dichlorométhane / méthanol / ammoniaque : 95/5/0,5)

Rendement : 19%

Microanalyse élémentaire :

| | C% | H% | N% |
|---|---|---|---|
| calculé : | 65,81 | 7,78 | 10,47 |
| trouvé : | 65,06 | 7,94 | 10,18 |

Résonance magnétique nucléaire du proton (DMSO d6) :

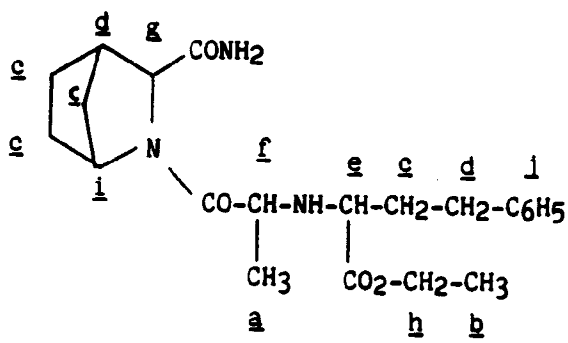

a δ = 1,1 ppm (3H,d)
b δ = 1,2 ppm (3H,t)
c δ entre 1,3 et 1,9 ppm (8H,m)
d δ = 2,6 ppm (3H,m)
e δ = 3,1 ppm (1H,m)
f δ = 3,6 ppm (1H,m)
g δ = 4,0 ppm (1H,m)
h δ = 4,1 ppm (2H,q)
i δ = 4,4 ppm (1H,m)
j δ = 7,2 ppm (5H,m)

**EXEMPLE 12 : [[ETHOXYCARBONYL-1 BUTYLAMINO-(S)]-2 PROPIONYL-(S)]-2 CARBAMOYL-3 AZA-2 BICYCLO [2.2.1]HEPTANE, ISOMERE α**

En procédant comme dans l'exemple 1, mais en remplaçant le carbamoyl-(S)-3 aza-2 bicyclo [2.2.2] octane par le carbamoyl-3 aza-2 bicyclo [2.2.1] heptane, isomère α décrit dans le brevet français n° 89.08672, on obtient le produit attendu.

Rendement : 38%

Microanalyse élémentaire :

| | C% | H% | N% |
|---|---|---|---|
| calculé : | 60,16 | 8,61 | 12,38 |
| trouvé : | 60,30 | 8,64 | 12,16 |

Résonance magnétique nucléaire du proton (DMSO d6) :

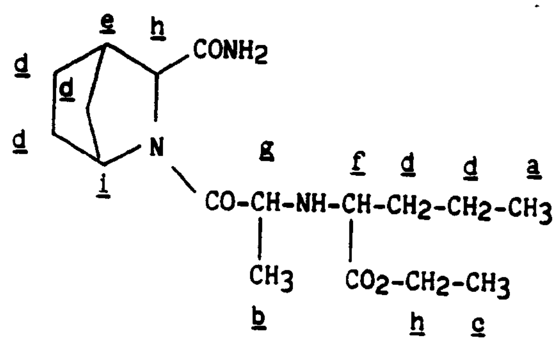

a δ = 0,8 ppm (3H,t)
b δ = 1,1 ppm (3H,d)
c δ = 1,2 ppm (3H,t)
d δ entre 1,0 et 1,7 ppm (10H,m)
e δ = 2,6 ppm (1H,m)
f δ = 3,1 ppm (1H,t)
g δ = 3,5 ppm (1H,q)
h δ = 4,1 ppm (3H,m)
i δ = 4,4 ppm (1H,m)

**EXEMPLE 13 : [[(ETHOXYCARBONYL)-1 BUTYLAMINO-(S)]-2 PROPIONYL-(S)]-2 CARBAMOYL-3-(S) DIMETHYL-1,4 AZA-2 BICYCLO [2.2.2] OCTANE**

En procédant comme dans l'exemple 1 mais en remplacant le carbamoyl-(S) aza-2 bicyclo [2.2.2] octane par le carbamoyl-(S) diméthyl-1,4 aza-2 bicyclo [2.2.2] octane décrit dans le brevet français n° 89 08672, on obtient le produit attendu.

**EXEMPLE 14 : ESTER n-OCTYLIQUE DE L'ACIDE [[CARBAMOYL-1 BUTYLAMINO-(S)]-2 PROPIONYL-(S)]-2 METHYL-4 ISOPROPYL-1 AZA-2 BICYCLO [2.2.2] OCTANE**

En procédant comme dans l'exemple 4 mais en remplacant au stade B l'acide aza-2 bicyclo [2.2.2] octanecarboxylique-3-(S) par l'acide méthyl-4 isopropyl-1 aza-2 bicyclo [2.2.2] octane décrit dans le brevet francais n° 89 08672, on obtient le produit attendu.

**ETUDE PHARMACOLOGIQUE DES DERIVES DE L'INVENTION**

**EXEMPLE 15 : EFFETS INHIBITEURS DE L'ENZYME DE CONVERSION DE L'ANGIOTENSINE I**

L'activité de l'enzyme de conversion est déterminée in vitro selon la méthode de RYAN J.W et al. (Biochem. J., 1977, 167, 501-504) qui utilise la réaction de transformation de l'hippuryl-histidyl-leucine (HHL) en acide hippurique. Ce dernier est dosé par scintillation liquide après extraction par l'acétate d'éthyle. L'effet inhibiteur d'un composé est déterminé par incubation à différentes doses en présence du substrat et de l'enzyme de conversion. Les résultats sont exprimés par l'IC50 de ces composés. Dans ces conditions le composé de

l'exemple 2 possède une IC50 égale à $5.10^{-8}$ M, celle du composé de l'exemple 8 étant de $1,8.10^{-8}$ M.

**EXEMPLE 16 : EFFETS PROMNESIQUES**

Des rats OFA (Iffa-Credo) mâles, de poids corporel 210-240 g sont placés en cages individuelles dans des conditions standard d'environnement. L'épreuve de mémorisation utilisée est une épreuve d'évitement passif à un essai dans laquelle l'amnésie expérimentale est provoquée par injection préalable de scopolamine. L'appareillage consiste en une enceinte à deux compartiments séparés par une porte à fermeture automatique.

Le jour de l'apprentissage, chaque animal est placé dans le compartiment aversif (blanc, fortement éclairé) qu'il peut explorer durant 60 secondes, temps au bout duquel il peut pénétrer librement dans le compartiment sécurisant (noir et obscur). Ce passage déclenche la fermeture de la porte puis l'application à l'animal d'un choc électrique par le plancher du compartiment sombre (0,6 mA; 3 secondes).

La rétention mnésique est mesurée 24 heures plus tard dans les mêmes conditions. Pour cela, la latence de passage vers le compartiment noir est mesurée et le temps d'observation maximum est fixé à 300 secondes. Dans ces conditions, les animaux témoins ne retournent pas dans le compartiment noir puni. Chez les animaux rendus amnésiques par l'injection de scopolamine (1 mg/kg IP) 30 minutes avant l'apprentissage, la latence de passage est fortement diminuée par rapport aux animaux témoins.

L'effet promnésique des composés étudiés est mesuré chez des animaux recevant la scopolamine. Ces composés sont administrés par voie IP, 2 fois par jour durant les 3 jours qui précèdent l'épreuve mnésique, puis 1 heure avant l'épreuve (soit 30 minutes avant scopolamine).
Les effets promnésiques des composés se manifestent par l'augmentation de la latence de passage par rapport aux animaux amnésiques.

Les composés de la présente invention sont comparés à l'oxiracétam, agent nootrope le plus récemment proposé dans la thérapeutique des troubles mnésiques.

Résultats :

Chez les animaux témoins non amnésiques, le temps moyen de latence de rétention est de 295 secondes et 95 % des animaux ne retournent jamais dans le compartiment puni.

Chez les animaux amnésiques, le temps moyen de latence de rétention est très significativement diminué (111,9 secondes; $p < 0.001$) et seulement 11% des animaux ne retournent pas dans le compartiment puni ($p < 0,005$).

Les composés de la présente invention s'opposent très significativement à l'amnésie induite par la scopolamine. Cet effet protecteur se manifeste pour certains d'entre eux dès la dose de 0,01 mg/kg et la gamme de doses promnésiques est alors de 0,01 à 1 mg/kg. Cet effet suit une courbe en U inversé caractéristique des composés facilitant les performances mnésiques.

Le tableau suivant regroupe pour exemple les résultats obtenus avec deux des dérivés de la présente invention et ceux obtenus avec le plus récent composé nootrope : l'oxiracetam. Dans ces conditions, celui-ci n'exerce une activité protectrice qu'à partir de 30 mg/kg.

| Doses en mg/kg | 0 | 0,003 | 0,01 | 0,03 | 0,1 | 0,3 | 1 | 3 |
|---|---|---|---|---|---|---|---|---|
| Exemple 3 Latence de passage (s) | 111,9 | 108,2 | 119,3 | 166,4 * | 171,6 * | 182,6 ** | 125,2 | 121,3 |
| % rétention à 300 sec. | 11 % | 5% | 10% | 35% ** | 45% *** | 40% *** | 20% | 10% |
| Exemple 4 Latence de passage (s) | 111,9 | 115,6 | 149,3 * | 205,1 *** | 207,2 *** | 202,1 *** | 171,5 ** | 106,7 |
| % rétention à 300 sec. | 11 % | 10% | 30% | 50% ** | 55% *** | 40% ** | 40% * | 5% |
| **Doses en mg/Kg.** | **0** | **1** | **3** | **10** | **30** | **100** | **300** | **1000** |
| OXIRACETAM Latence de passage (s) | 111,9 | 97,4 | 126,5 | 115,3 | 150,1 * | 182,5 * | 200,2 *** | 175,8 * |
| % rétention à 300 sec. | 11 % | 10% | 10% | 5% | 30% * | 40% ** | 45% ** | 30% |

* p<0,05 ** p<0,01 *** p<0,005

vs témoins scopolamine

Latence de passage : Test de Mann-Whitney

% rétention300 sec : Test du Log-rank sur les évolutions des % d'évitement lors des 300 secondes de la phase de rétention

Ainsi l'effet nootrope des composés de la présente invention s'exerce notamment par le biais de la neurotransmission cholinergique qu'ils facilitent pour améliorer les capacités de mémorisation et de remémoration.

**EXEMPLE 17 : COMPOSITION PHARMACEUTIQUE**

Comprimé formule de préparation pour 1000 comprimés dosés à 2 mg de principe actif.

| | |
|---|---|
| [[Ethoxycarbonyl-1 butylamino-(S)]-2 propionyl-(S)]-2 carbamoyl-3-(S) aza-2 bicyclo [2.2.2] octane | 2 g |
| Hydroxy propyl cellulose | 2 g |
| Amidon de blé | 10 g |
| Lactose | 100 g |
| Stéarate de magnésium | 3 g |
| Talc | 3 g |

## Revendications

### Revendications pour les Etats contractants suivants : AT, BE, CH, DE, DK, FR, GB, IT, LI, LU, NL, SE

**1.** Composés de formule générale (I) :

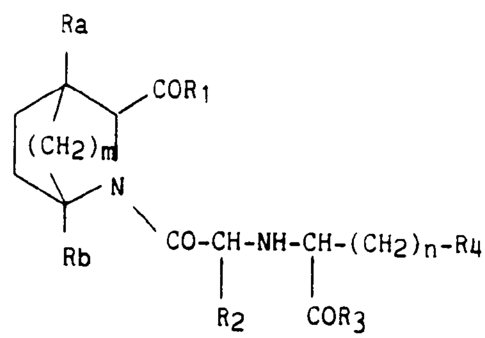

(I)

dans laquelle :

- $R_1$ représente un groupement alcoxy inférieur ou supérieur linéaire ou ramifié, ou un groupement amino éventuellement substitué par un ou deux groupements alkyle inférieur linéaire ou ramifié,
- $R_2$ représente un groupement alkyle inférieur linéaire ou ramifié éventuellement substitué par un groupement amino,
- $R_3$ représente un groupement amino, un groupement alcoxy inférieur ou supérieur linéaire ou ramifié, un groupement hydroxyle, à condition toutefois qu'au moins un groupement amino soit présent en $R_1$ ou $R_3$,
- $R_4$ représente un atome d'hydrogène ou un groupement aryle,
- m est égal à 1 ou 2,
- n est compris entre 1 et 6,
- Ra et Rb identiques ou différents représentent quand m = 1, un atome d'hydrogène, quand m = 2, un groupement alkyle inférieur linéaire ou ramifié ou un atome d'hydrogène,

  le terme inférieur indiquant que les groupements ainsi qualifiés comptent de 1 à 6 atomes de carbone, le terme supérieur indiquant que les groupements ainsi qualifiés comptent de 7 à 12 atomes de carbone,

leurs énantiomères, diastéréoisomères et épimères ainsi que leurs sels d'addition à un acide ou à une base pharmaceutiquement acceptable.

**2.** Composés selon la revendication 1 tels que m est égal à 1.

**3.** Composés selon la revendication 1 tels que m est égal à 2.

**4.** Composés selon la revendication 1 tels que $R_1$ représente un groupement amino.

5. Composés selon la revendication 1 tels que R1 représente un groupement n-octyl et R3 représente un groupement amino

6. Procédé de préparation des dérivés de formule (I) caractérisé en ce que l'on soumet à une amination réductrice le composé de formule (II) :

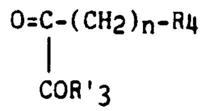

(II)

dans laquelle $R_4$ a la même signification que dans la formule (I), et $R'_3$ représente un groupement amino ou alcoxy inférieur ou supérieur, linéaire ou ramifié, en présence d'une hydrure mixte de métal alcalin tel que le cyanoborohydrure de sodium avec un amino acide de formule (III) dont la fonction acide est protégée :

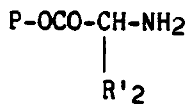

(III)

dans laquelle P est un groupement alkyle tel que le tertbutyl et $R'_2$ un groupement alkyle inférieur linéaire ou ramifié éventuellement substitué par un groupement amino lui-même protégé par un groupement protecteur tel que le benzyloxycarbonyle,
pour obtenir un dérivé de formule (IV) :

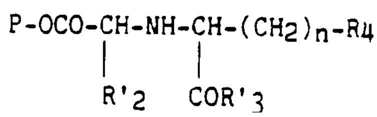

(IV)

dans laquelle $R'_2$, $R'_3$, $R_4$, P et n ont les mêmes significations que précédemment, dont on sépare éventuellement les isomères par une technique classique de séparation et que l'on déprotège en milieu acide pour obtenir un dérivé de formule (V) :

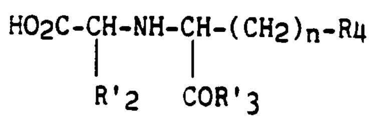

(V)

dans laquelle $R'_2$, $R'_3$, $R_4$ et n ont les mêmes significations que précédemment,
qui est alors couplé avec un dérivé de formule (VI) :

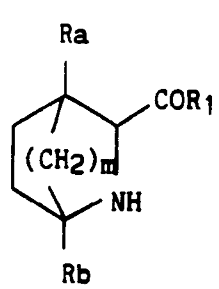

(VI)

dans laquelle Ra, Rb, m et $R_1$ ont les mêmes significations que dans la formule (I),
pour conduire à un dérivé de formule (I/a) cas particulier des dérivés de formule (I) :

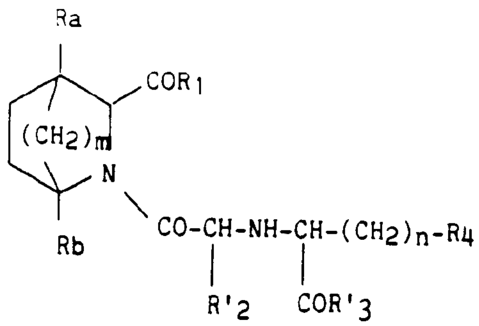

(I/a)

dans laquelle Ra, Rb, $R_1$, $R'_2$, $R'_3$, $R_4$, m et n ont la même signification que précédemment, qui, si on le désire, lorsque $R'_2$ représente un groupement alkyle inférieur linéaire ou ramifié et $R'_3$ est un groupement alcoxy inférieur ou supérieur linéaire ou ramifié peut être soumis à l'action d'une base ou d'un acide pour conduire à un dérivé de formule (I/b), cas particulier des dérivés de formule (I) :

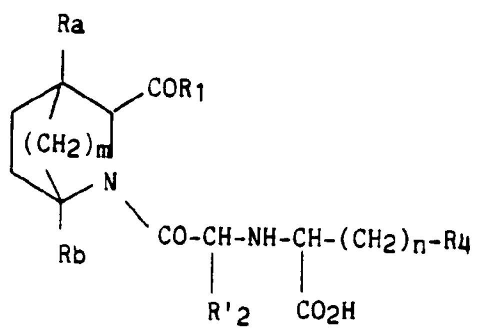

(I/b)

dans laquelle Ra, Rb, $R_1$, $R_4$, m, n ont la même signification que dans la formule (I), $R'_2$ représente un groupement alkyle inférieur linéaire ou ramifié et $R_3$ représente un groupement hydroxyle, ou bien, lorsque $R'_2$ est un groupement alkyle substitué par un groupement amino protégé et $R'_3$ un groupement alcoxy inférieur ou supérieur, linéaire ou ramifié, soumis à une hydrogénation en présence d'un catalyseur tel que le charbon palladié, pour conduire à un dérivé de formule (I/c), cas particulier des dérivés de formule (I) :

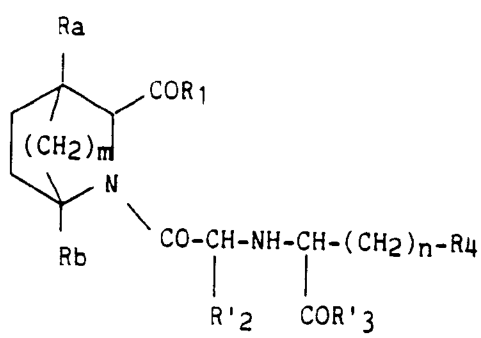

(I/c)

dans laquelle Ra, Rb, $R_1$, $R_4$, n et m ont les mêmes significations que dans la formule (I), $R'_2$ représente un groupement alkyle substitué par un groupement amino et $R'_3$ représente un groupement alcoxy inférieur ou supérieur linéaire ou ramifié, dérivé (I/c) qui, si on le désire, peut être soumis à l'action d'une base, pour conduire à un dérivé de formule (I/d), cas particulier des dérivés de formule (I) :

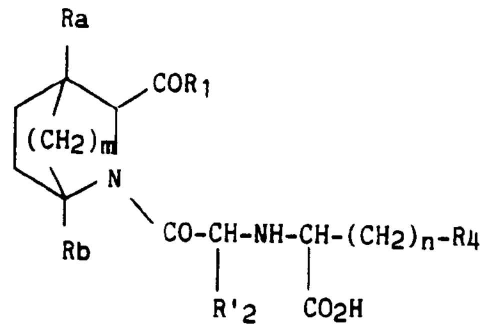

(I/d)

dans laquelle Ra, Rb, $R_1$, $R_4$, n et m ont les mêmes significations que dans la formule (I), $R'_2$ représente un groupement alkyle substitué par un groupement amino et $R_3$ représente un groupement hydroxyle, dérivés de formule (I/a), (I,b), (I/c) et (I/d) que l'on purifie par une technique classique de purification, dont on sépare éventuellement les isomères par une technique classique de séparation et que l'on transforme, si on le désire, en leurs sels d'addition à un acide ou à une base pharmaceutiquement acceptable.

**7.** Composés de formule (VI) dans laquelle $R_1$ représente un groupement alcoxy supérieur utiles dans la préparation des composés de formule (I).

**8.** Compositions pharmaceutiques contenant comme principe actif au moins un composé selon l'une quelconque des revendication 1 à 5 seul ou en combinaison avec un ou plusieurs excipients ou véhicules inertes, non toxiques, pharmaceutiquement acceptables.

**9.** Compositions pharmaceutiques selon la revendication 8 contenant au moins un principe actif selon l'une des revendications 1 à 5 utiles pour le traitement de l'hypertension artérielle et des troubles neurocomportementaux associés au vieillissement et aux démences dégénératives séniles ou préséniles telles que la maladie d'Alzheimer, la maladie de Pick, la démence multi-infarctus et la maladie de Binswanger.

**Revendications pour l'Etat contractant suivant : GR**

**1.** Procédé de préparation des composés de formule générale (I) :

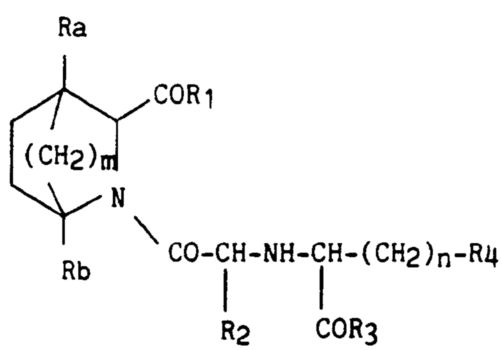

(I)

dans laquelle :

- $R_1$ représente un groupement alcoxy inférieur ou supérieur linéaire ou ramifié, ou un groupement amino éventuellement substitué par un ou deux groupements alkyle inférieur linéaire ou ramifié,
- $R_2$ représente un groupement alkyle inférieur linéaire ou ramifié éventuellement substitué par un groupement amino,
- $R_3$ représente un groupement amino, un groupement alcoxy inférieur ou supérieur linéaire ou ramifié, un groupement hydroxyle, à condition toutefois qu'au moins un groupement amino soit présent en $R_1$ ou $R_3$,
- $R_4$ représente un atome d'hydrogène ou un groupement aryle,
- m est égal à 1 ou 2,
- n est compris entre 1 et 6,

- Ra et Rb identiques ou différents représentent quand m = 1, un atome d'hydrogène, quand m = 2, un groupement alkyle inférieur linéaire ou ramifié ou un atome d'hydrogène,
  le terme inférieur indiquant que les groupements ainsi qualifiés comptent de 1 à 6 atomes de carbone,
  le terme supérieur indiquant que les groupements ainsi qualifiés comptent de 7 à 12 atomes de carbone,

leurs énantiomères, diastéréoisomères et épimères ainsi que leurs sels d'addition à un acide ou à une base pharmaceutiquement acceptable,
caractérisé en ce que l'on soumet à une amination réductrice le composé de formule (II) :

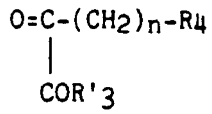

(II)

dans laquelle $R_4$ a la même signification que dans la formule (I), et $R'_3$ représente un groupement amino ou alcoxy inférieur ou supérieur, linéaire ou ramifié, en présence d'une hydrure mixte de métal alcalin tel que le cyanoborohydrure de sodium avec un amino acide de formule (III) dont la fonction acide est protégée :

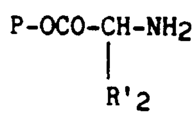

(III)

dans laquelle P est un groupement alkyle tel que le tertbutyl et $R'_2$ un groupement alkyle inférieur linéaire ou ramifié éventuellement substitué par un groupement amino lui-même protégé par un groupement protecteur tel que le benzyloxycarbonyle,
pour obtenir un dérivé de formule (IV) :

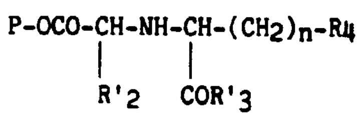

(IV)

dans laquelle $R'_2$, $R'_3$, $R_4$, P et n ont les mêmes significations que précédemment, dont on sépare éventuellement les isomères par une technique classique de séparation et que l'on déprotège en milieu acide pour obtenir un dérivé de formule (V) :

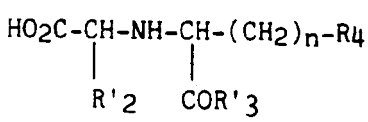

(V)

dans laquelle $R'_2$, $R'_3$, $R_4$ et n ont les mêmes significations que précédemment,
qui est alors couplé avec un dérivé de formule (VI) :

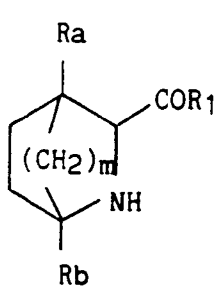

(VI)

dans laquelle Ra, Rb, m et $R_1$ ont les mêmes significations que dans la formule (I), pour conduire à un dérivé de formule (I/a) cas particulier des dérivés de formule (I) :

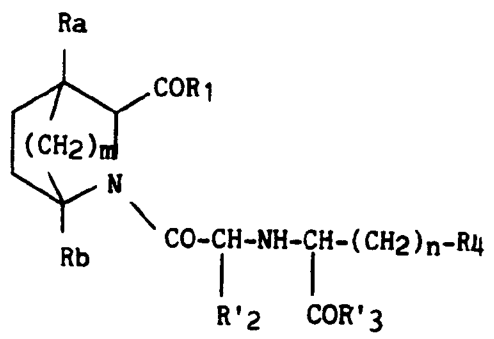

(I/a)

dans laquelle Ra, Rb, $R_1$, $R'_2$, $R'_3$, $R_4$, m et n ont la même signification que précédemment, qui, si on le désire, lorsque $R'_2$ représente un groupement alkyle inférieur linéaire ou ramifié et $R'_3$ est un groupement alcoxy inférieur ou supérieur linéaire ou ramifié peut être soumis à l'action d'une base ou d'un acide pour conduire à un dérivé de formule (I/b), cas particulier des dérivés de formule (I) :

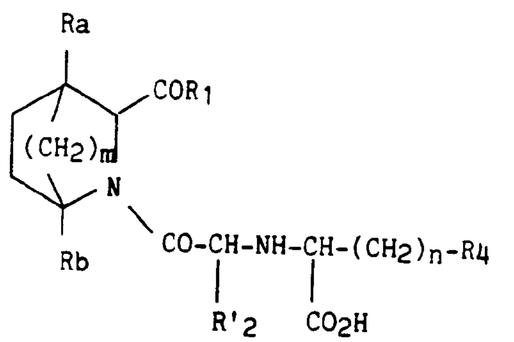

(I/b)

dans laquelle Ra, Rb, $R_1$, $R_4$, m, n ont la même signification que dans la formule (I), $R'_2$ représente un groupement alkyle inférieur linéaire ou ramifié et $R_3$ représente un groupement hydroxyle, ou bien, lorsque $R'_2$ est un groupement alkyle substitué par un groupement amino protégé et $R'_3$ un groupement alcoxy inférieur ou supérieur, linéaire ou ramifié, soumis à une hydrogénation en présence d'un catalyseur tel que le charbon palladié, pour conduire à un dérivé de formule (I/c), cas particulier des dérivés de formule (I) :

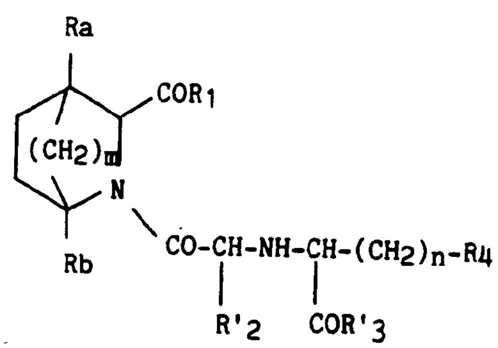

(I/c)

dans laquelle Ra, Rb, $R_1$, $R_4$, n et m ont les mêmes significations que dans la formule (I), $R'_2$ représente un groupement alkyle substitué par un groupement amino et $R'_3$ représente un groupement alcoxy inférieur ou supérieur linéaire ou ramifié,
dérivé (I/c) qui, si on le désire, peut être soumis à l'action d'une base, pour conduire à un dérivé de formule

(I/d), cas particulier des dérivés de formule (I) :

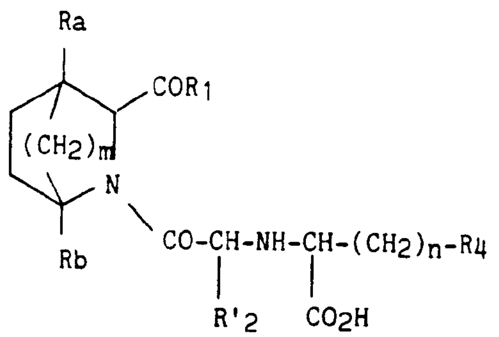

(I/d)

dans laquelle Ra, Rb, $R_1$, $R_4$, n et m ont les mêmes significations que dans la formule (I), $R'_2$ représente un groupement alkyle substitué par un groupement amino et $R_3$ représente un groupement hydroxyle, dérivés de formule (I/a), (I,b), (I/c) et (I/d) que l'on purifie par une technique classique de purification, dont on sépare éventuellement les isomères par une technique classique de séparation et que l'on transforme, si on le désire, en leurs sels d'addition à un acide ou à une base pharmaceutiquement acceptable.

2. Procédé de préparation selon la revendication 1 des composés tels que m est égal à 1.

3. Procédé de préparation selon la revendication 1 des composés tels que m est égal à 2.

4. Procédé de préparation selon la revendication 1 des composés tels que $R_1$ représente un groupement amino.

5. Procédé de préparation selon la revendication 1 des composés tels que $R_1$ représente un groupement n-octyl et $R_3$ représente un groupement amino

6. Procédé de préparation selon la revendication 1 des composés de formule (VI) dans laquelle $R_1$ représente un groupement alcoxy supérieur utiles dans la préparation des composés de formule (I).

**Revendications pour l'Etat contractant suivant : ES**

1. Procédé de préparation des composés de formule générale (I) :

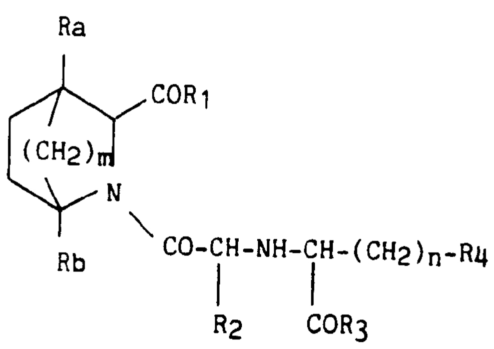

(I)

dans laquelle :

- $R_1$ représente un groupement alcoxy inférieur ou supérieur linéaire ou ramifié, ou un groupement amino éventuellement substitué par un ou deux groupements alkyle inférieur linéaire ou ramifié,
- $R_2$ représente un groupement alkyle inférieur linéaire ou ramifié éventuellement substitué par un groupement amino,
- $R_3$ représente un groupement amino, un groupement alcoxy inférieur ou supérieur linéaire ou ramifié,

un groupement hydroxyle, à condition toutefois qu'au moins un groupement amino soit présent en $R_1$ ou $R_3$,

- $R_4$ représente un atome d'hydrogène ou un groupement aryle,
- m est égal à 1 ou 2,
- n est compris entre 1 et 6,
- Ra et Rb identiques ou différents représentent quand m = 1, un atome d'hydrogène, quand m = 2, un groupement alkyle inférieur linéaire ou ramifié ou un atome d'hydrogène,

  le terme inférieur indiquant que les groupements ainsi qualifiés comptent de 1 à 6 atomes de carbone, le terme supérieur indiquant que les groupements ainsi qualifiés comptent de 7 à 12 atomes de carbone,

leurs énantiomères, diastéréoisomères et épimères ainsi que leurs sels d'addition à un acide ou à une base pharmaceutiquement acceptable,
caractérisé en ce que l'on soumet à une amination réductrice le composé de formule (II) :

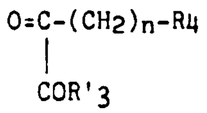
(II)

dans laquelle $R_4$ a la même signification que dans la formule (I), et $R'_3$ représente un groupement amino ou alcoxy inférieur ou supérieur, linéaire ou ramifié, en présence d'une hydrure mixte de métal alcalin tel que le cyanoborohydrure de sodium avec un amino acide de formule (III) dont la fonction acide est protégée :

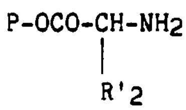
(III)

dans laquelle P est un groupement alkyle tel que le tertbutyl et $R'_2$ un groupement alkyle inférieur linéaire ou ramifié éventuellement substitué par un groupement amino lui-même protégé par un groupement protecteur tel que le benzyloxycarbonyle,
pour obtenir un dérivé de formule (IV) :

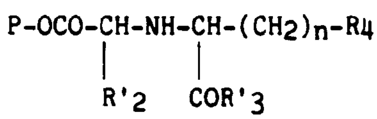
(IV)

dans laquelle $R'_2$, $R'_3$, $R_4$, P et n ont les mêmes significations que précédemment, dont on sépare éventuellement les isomères par une technique classique de séparation et que l'on déprotège en milieu acide pour obtenir un derivé de formule (V) :

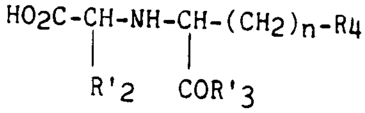
(V)

dans laquelle $R'_2$, $R'_3$, $R_4$ et n ont les mêmes significations que précédemment,
qui est alors couplé avec un dérivé de formule (VI) :

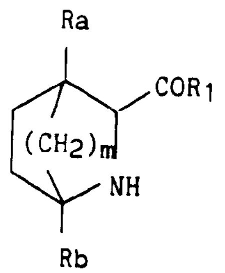

(VI)

dans laquelle Ra, Rb, m et $R_1$ ont les mêmes significations que dans la formule (I), pour conduire à un dérivé de formule (I/a) cas particulier des dérivés de formule (I) :

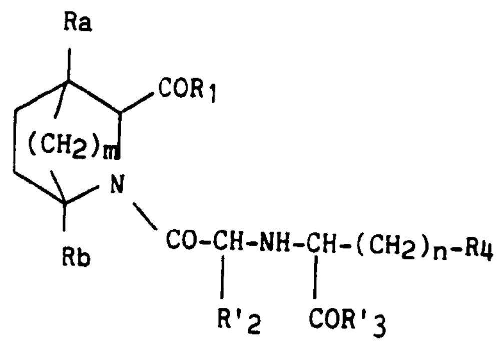

(I/a)

dans laquelle Ra, Rb, $R_1$, $R'_2$, $R'_3$, $R_4$, m et n ont la même signification que précédemment, qui, si on le désire, lorsque $R'_2$ représente un groupement alkyle inférieur linéaire ou ramifié et $R'_3$ est un groupement alcoxy inférieur ou supérieur linéaire ou ramifié peut être soumis à l'action d'une base ou d'un acide pour conduire à un dérivé de formule (I/b), cas particulier des dérivés de formule (I) :

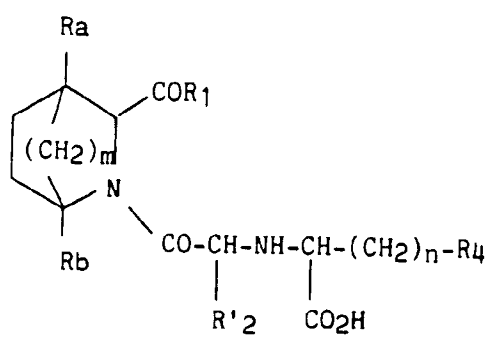

(I/b)

dans laquelle Ra, Rb, $R_1$, $R_4$, m, n ont la même signification que dans la formule (I), $R'_2$ représente un groupement alkyle inférieur linéaire ou ramifié et $R_3$ représente un groupement hydroxyle, ou bien, lorsque $R'_2$ est un groupement alkyle substitué par un groupement amino protégé et $R'_3$ un groupement alcoxy inférieur ou supérieur, linéaire ou ramifié, soumis à une hydrogénation en présence d'un catalyseur tel que le charbon palladié, pour conduire à un dérivé de formule (I/c), cas particulier des dérivés de formule (I) :

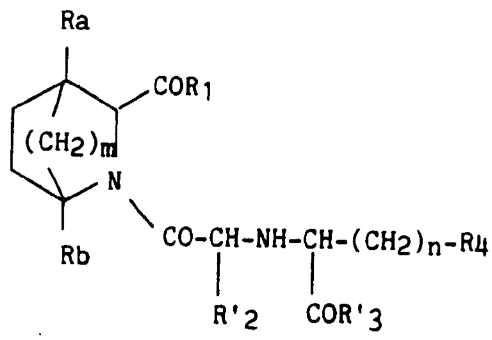

(I/c)

dans laquelle Ra, Rb, $R_1$ , $R_4$, n et m ont les mêmes significations que dans la formule (I), $R'_2$ représente un groupement alkyle substitué par un groupement amino et $R'_3$ représente un groupement alcoxy inférieur ou supérieur linéaire ou ramifié,
dérivé (I/c) qui, si on le désire, peut être soumis à l'action d'une base, pour conduire à un dérivé de formule (I/d), cas particulier des dérivés de formule (I) :

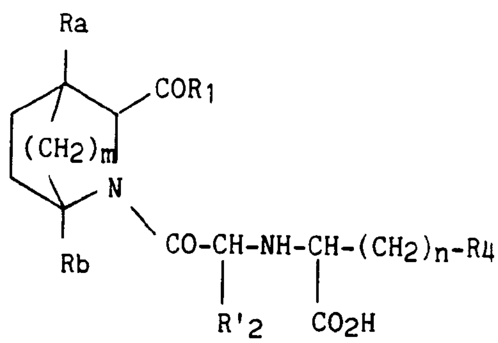

(I/d)

dans laquelle Ra, Rb, $R_1$, $R_4$, n et m ont les mêmes significations que dans la formule (I), $R'_2$ représente un groupement alkyle substitué par un groupement amino et $R_3$ représente un groupement hydroxyle, dérivés de formule (I/a), (I,b), (I/c) et (I/d) que l'on purifie par une technique classique de purification, dont on sépare éventuellement les isomères par une technique classique de séparation et que l'on transforme, si on le désire, en leurs sels d'addition à un acide ou à une base pharmaceutiquement acceptable.

2. Procédé de préparation selon la revendication 1 des composés tels que m est égal à 1.

3. Procédé de préparation selon la revendication 1 des composés tels que m est égal à 2.

4. Procédé de préparation selon la revendication 1 des composés tels que $R_1$ représente un groupement amino.

5. Procédé de préparation selon la revendication 1 des composés tels que $R_1$ représente un groupement n-octyl et $R_3$ représente un groupement amino

6. Procédé de préparation selon la revendication 1 des composés de formule (VI) dans laquelle $R_1$ représente un groupement alcoxy supérieur utiles dans la préparation des composés de formule (I).

**Patentansprüche**

**Patentansprüche für folgende Vertragsstaaten : AT, BE, CH, DE, DK, FR, GB, IT, LI, LU, NL, SE**

1. Verbindungen der allgemeinen Formel (I):

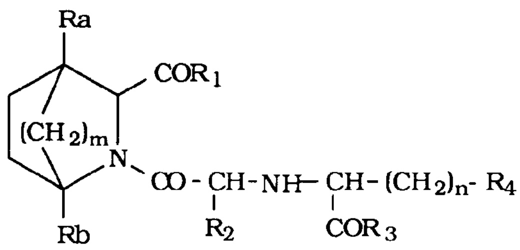

(I)

in der :

- $R_1$ eine geradkettige oder verzweigte niedrigmolekulare oder höhermolekulare Alkoxygruppe oder eine Aminogruppe, die gegebenenfalls durch eine oder zwei geradkettige oder verzweigte niedrigmolekulare Alkylgruppen substituiert ist,
- $R_2$ eine geradkettige oder verzweigte niedrigmolekulare Alkylgruppe, die gegebenenfalls durch eine Aminogruppe substituiert ist,
- $R_3$ eine Aminogruppe, eine geradkettige oder verzweigte niedrigmolekulare oder höhermolekulare Alkoxygruppe, eine Hydroxylgruppe, mit der Maßgabe, daß in $R_1$ oder $R_3$ mindestens eine Aminogruppe vorhanden ist,
- $R_4$ ein Wasserstoffatom oder eine Arylgruppe,
- m 1 oder 2,
- n zwischen 1 und 6,
- Ra und Rb, die gleichartig oder verschieden sein können, wenn m = 1, ein Wasserstoffatom, wenn m = 2, eine geradkettige oder verzweigte niedrigmolekulare Alkylgruppe oder ein Wasserstoffatom bedeuten, wobei der Begriff niedrigmolekular bedeutet, daß die in dieser Weise bezeichneten Gruppen 1 bis 6 Kohlenstoffatome aufweisen, wobei der Begriff höhermolekular bedeutet, daß die in dieser Weise bezeichneten Gruppen 7 bis 12 Kohlenstoffatome aufweisen,

deren Enantiomere, Diastereoisomere und Epimere sowie deren Additionssalze mit einer pharmazeutisch annehmbaren Säure oder Base.

2. Verbindungen nach Anspruch 1, worin m 1 bedeutet.

3. Verbindungen nach Anspruch 1, worin m 2 bedeutet.

4. Verbindungen nach Anspruch 1, worin $R_1$ eine Aminogruppe bedeutet.

5. Verbindungen nach Anspruch 1, worin $R_1$ eine n-Octylgruppe und $R_3$ eine Aminogruppe bedeuten.

6. Verfahren zur Herstellung der Derivate der Formel (I), **dadurch gekennzeichnet,** daß man die Verbindung der Formel (II):

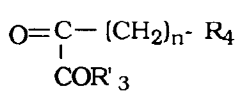

(II)

in der $R_4$ die bezüglich der Formel (I) angegebenen Bedeutungen besitzt und $R'_3$ eine Aminogruppe oder eine geradkettige oder verzweigte niedrigmolekulare oder höhermolekulare Alkoxygruppe bedeutet, in Gegenwart eines gemischten Alkalimetallhydrids, wie Natriumcyanoborhydrid, mit einer Aminosäure der Formel (III), deren Säuregruppe geschützt ist:

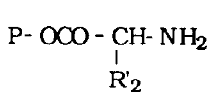

(III)

in der P eine Alkylgruppe, wie eine tert.-Butylgruppe, und $R'_2$ eine geradkettige oder verzweigte niedrigmolekulare Alkylgruppe, die gegebenenfalls durch eine Aminosäure substituiert ist, die ihrerseits durch eine Schutzgruppe, wie die Benzyloxycarbonylgruppe, geschützt ist, einer reduzierenden Aminierung unterwirft,
so daß man ein Derivat der Formel (IV) erhält:

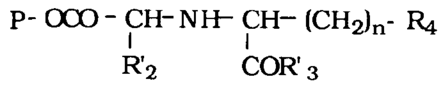

(IV)

in der $R'_2$, $R'_3$, $R_4$, P und n die oben angegebenen Bedeutungen besitzen, deren Isomere man gegebenenfalls mit Hilfe einer klassischen Trennmethode trennt und von dem man die Schutzgruppen in einem sauren Medium abspaltet, so daß man ein Derivat der Formel (V) erhält:

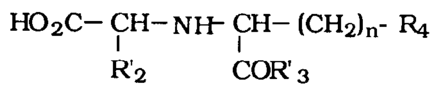

(V)

in der $R'_2$, $R'_3$, $R_4$ und n die oben angegebenen Bedeutungen besitzen, welches dann mit einem Derivat der Formel (VI):

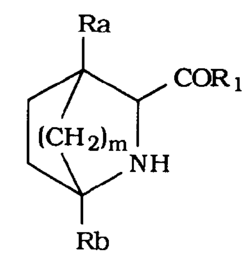

(VI)

in der Ra, Rb, m und $R_1$ die bezüglich der Formel (I) angegebenen Bedeutungen besitzen, kuppelt,
so daß man ein Derivat der Formel (I/a) erhält, einem Sonderfall der Derivate der Formel (I):

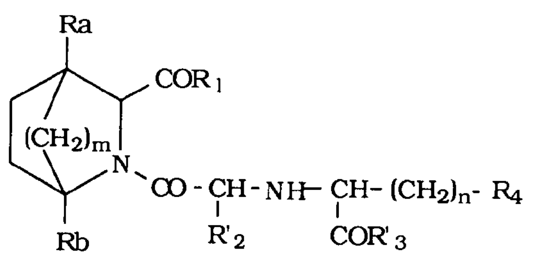

(I/a)

worin Ra, Rb, $R_1$, $R'_2$, $R'_3$, $R_4$, m und n die oben angegebenen Bedeutungen besitzen,
welches man gewünschtenfalls, wenn $R'_2$ eine geradkettige oder verzweigte niedrigmolekulare Alkylgruppe und $R'_3$ eine geradkettige oder verzweigte niedrigmolekulare oder höhermolekulare Alkoxygruppe bedeuten, der Einwirkung einer Base oder einer Säure unterwirft, so daß man ein Derivat der Formel (I/b) erhält, einem Sonderfall der Derivate der Formel (I) :

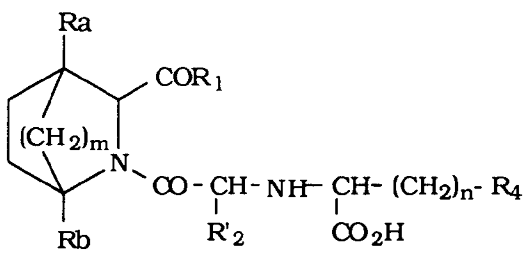

(I/b)

in der Ra, Rb, $R_1$, $R_4$, m und n die bezüglich der Formel (I) angegebenen Bedeutungen besitzen, $R'_2$ eine geradkettige oder verzweigte niedrigmolekulare Alkylgruppe und $R_3$ eine Hydroxylgruppe bedeuten, oder, wenn $R'_2$ eine durch eine geschützte Aminogruppe substituierte Alkylgruppe und $R'_3$ eine geradkettige oder verzweigte niedrigmolekulare oder höhermolekulare Alkoxygruppe bedeuten, einer Hydrierung in Gegenwart eines Katalysators, wie Palladium-auf-Kohlenstoff, unterwirft, so daß man ein Derivat der Formel (I/c) erhält, einem Sonderfall der Derivate der Formel (I):

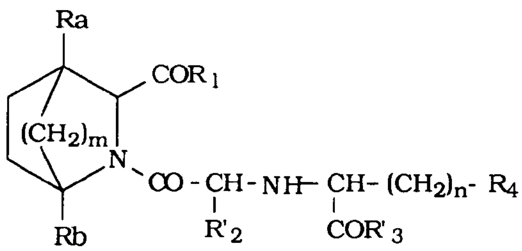

(I/c)

worin Ra, Rb, $R_1$, $R_4$, n und m die bezüglich der Formel (I) angegebenen Bedeutungen besitzen, $R'_2$ eine durch eine Aminogruppe substituierte Alkylgruppe und $R'_3$ eine geradkettige oder verzweigte niedrigmolekulare oder höhermolekulare Alkoxygruppe bedeuten,
welches Derivat (I/c) gewünschtenfalls der Einwirkung einer Base unterworfen werden kann, so daß man ein Derivat der Formel (I/d) erhält, einem Sonderfall der Derivate der Formel (I):

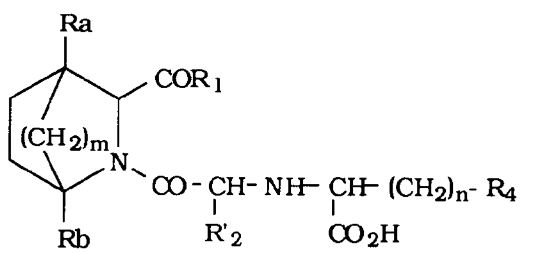

(I/d)

in der Ra, Rb, $R_1$, $R_4$, n und m die bezüglich der Formel (I) angegebenen Bedeutungen besitzen, $R'_2$ eine durch eine Aminogruppe substituierte Alkylgruppe und $R_3$ eine Hydroxylgruppe bedeuten,
welche Derivate der Formel (I/a), (I/b), (I/c) und (I/d) man mit Hilfe einer klassischen Reinigungsmethode reinigen kann und die man gegebenenfalls mit Hilfe einer klassischen Trennmethode in die Isomeren trennen kann und die man gewünschtenfalls mit einer pharmazeutisch annehmbaren Säure oder Base in ihre Säureadditionssalze umwandeln kann.

7. Verbindungen der Formel (VI), worin $R_1$ eine höhermolekulare Alkoxygruppe bedeutet, zur Herstellung von Verbindungen der Formel (I).

8. Pharmazeutische Zubereitungen enthaltend als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien oder Bindemitteln.

9. Pharmazeutische Zubereitungen nach Anspruch 8, enthaltend mindestens einen Wirkstoff nach einem der Ansprüche 1 bis 5 zur Behandlung der arteriellen Hypertension oder von Nervenverhaltensstörungen, die mit dem Altern und senilen oder präsenilen degenerativen Dementien verknüpft sind, wie der Alzheimer'schen Krankheit, der Pick'schen Krankheit, der Multiinfarkt-Demenz und der Binswanger Krankheit.

**Patentansprüche für folgenden Vertragsstaat : GR**

1. Verfahren zur Herstellung von Verbindungen der allgemeinen Formel (I):

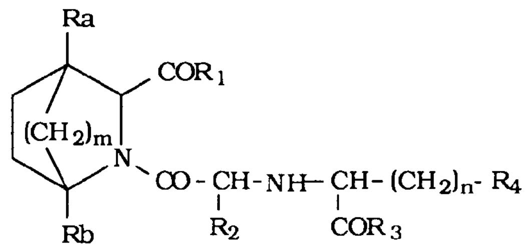

(I)

in der:
- $R_1$ eine geradkettige oder verzweigte niedrigmolekulare oder höhermolekulare Alkoxygruppe oder eine Aminogruppe, die gegebenenfalls durch eine oder zwei geradkettige oder verzweigte niedrigmolekulare Alkylgruppen substituiert ist,
- $R_2$ eine geradkettige oder verzweigte niedrigmolekulare Alkylgruppe, die gegebenenfalls durch eine Aminogruppe substituiert ist,
- $R_3$ eine Aminogruppe, eine geradkettige oder verzweigte niedrigmolekulare oder höhermolekulare Alkoxygruppe, eine Hydroxylgruppe, mit der Maßgabe, daß in $R_1$ oder $R_3$ mindestens eine Aminogruppe vorhanden ist,
- $R_4$ ein Wasserstoffatom oder eine Arylgruppe,
- m 1 oder 2,
- n zwischen 1 und 6,
- Ra und Rb, die gleichartig oder verschieden sein können, wenn m = 1, ein Wasserstoffatom, wenn m = 2, eine geradkettige oder verzweigte niedrigmolekulare Alkylgruppe oder ein Wasserstoffatom bedeuten,
  wobei der Begriff niedrigmolekular bedeutet, daß die in dieser Weise bezeichneten Gruppen 1 bis 6 Kohlenstoffatome aufweisen,
  wobei der Begriff höhermolekular bedeutet, daß die in dieser Weise bezeichneten Gruppen 7 bis 12 Kohlenstoffatome aufweisen,

sowie von deren Enantiomeren, Diastereoisomeren und Epimeren sowie von deren Additionssalzen mit einer pharmazeutisch annehmbaren Säure oder Base, **dadurch gekennzeichnet,** daß man die Verbindung der Formel (II):

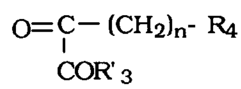

(II)

in der $R_4$ die bezüglich der Formel (I) angegebenen Bedeutungen besitzt und $R'_3$ eine Aminogruppe oder eine geradkettige oder verzweigte niedrigmolekulare oder höhermolekulare Alkoxygruppe bedeutet. in Gegenwart eines gemischten Alkalimetallhydrids, wie Natriumcyanoborhydrid, mit einer Aminosäure der Formel (III), deren Säuregruppe geschützt ist:

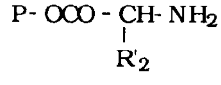

(III)

in der P eine Alkylgruppe, wie eine tert.-Butylgruppe, und $R'_2$ eine geradkettige oder verzweigte niedrigmolekulare Alkylgruppe, die gegebenenfalls durch eine Aminosäure substituiert ist, die ihrerseits durch eine Schutzgruppe, wie die Benzyloxycarbonylgruppe, geschützt ist, einer reduzierenden Aminierung unterwirft,
so daß man ein Derivat der Formel (IV) erhält:

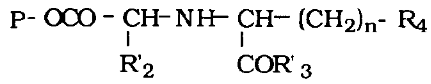

(IV)

in der $R'_2$, $R'_3$, $R_4$, P und n die oben angegebenen Bedeutungen besitzen, deren Isomere man gegebenenfalls mit Hilfe einer klassischen Trennmethode trennt und von dem man die Schutzgruppen in einem sauren Medium abspaltet, so daß man ein Derivat der Formel (V) erhält:

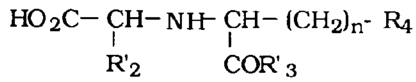

(V)

in der $R'_2$, $R'_3$, $R_4$ und n die oben angegebenen Bedeutungen besitzen, welches dann mit einem Derivat der Formel (VI):

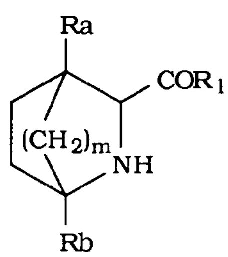

(VI)

in der Ra, Rb, m und $R_1$ die bezüglich der Formel (I) angegebenen Bedeutungen besitzen, kuppelt,
so daß man ein Derivat der Formel (I/a) erhält, einem Sonderfall der Derivate der Formel (I):

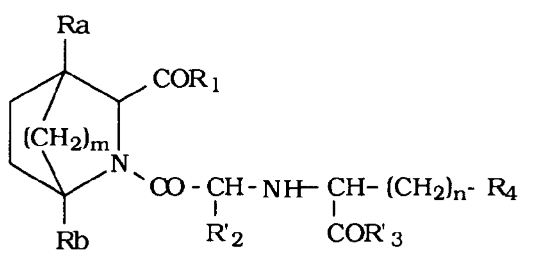

(I/a)

worin Ra, Rb, $R_1$, $R'_2$, $R'_3$, $R_4$, m und n die oben angegebenen Bedeutungen besitzen,
welches man gewünschtenfalls, wenn $R'_2$ eine geradkettige oder verzweigte niedrigmolekulare Alkylgruppe und $R'_3$ eine geradkettige oder verzweigte niedrigmolekulare oder höhermolekulare Alkoxygruppe bedeuten, der Einwirkung einer Base oder einer Säure unterwirft, so daß man ein Derivat der Formel (I/b) erhält, einem Sonderfall der Derivate der Formel (I):

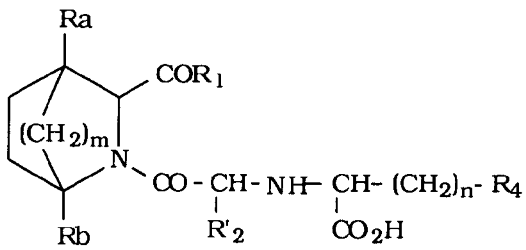

(I/b)

in der Ra, Rb, $R_1$, $R_4$, m und n die bezüglich der Formel (I) angegebenen Bedeutungen besitzen, $R'_2$ eine geradkettige oder verzweigte niedrigmolekulare Alkylgruppe und $R_3$ eine Hydroxylgruppe bedeuten, oder, wenn $R'_2$ eine durch eine geschützte Aminogruppe substituierte Alkylgruppe und $R'_3$ eine geradkettige oder verzweigte niedrigmolekulare oder höhermolekulare Alkoxygruppe bedeuten, einer Hydrierung in Gegenwart eines Katalysators, wie Palladium-auf-Kohlenstoff, unterwirft, so daß man ein Derivat der Formel (I/c) erhält, einem Sonderfall der Derivate der Formel (I):

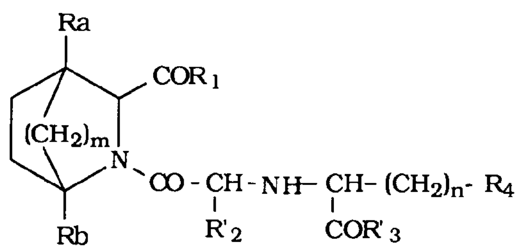

(I/c)

worin Ra, Rb, $R_1$, $R_4$, n und m die bezüglich der Formel (I) angegebenen Bedeutungen besitzen, $R'_2$ eine durch eine Aminogruppe substituierte Alkylgruppe und $R'_3$ eine geradkettige oder verzweigte niedrigmolekulare oder höhermolekulare Alkoxygruppe bedeuten,
welches Derivat (I/c) gewünschtenfalls der Einwirkung einer Base unterworfen werden kann, so daß man ein Derivat der Formel (I/d) erhält, einem Sonderfall der Derivate der Formel (I):

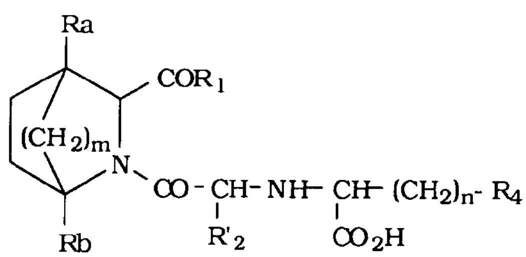

(I/d)

in der Ra, Rb, $R_1$, $R_4$, n und m die bezüglich der Formel (I) angegebenen Bedeutungen besitzen, $R'_2$ eine durch eine Aminogruppe substituierte Alkylgruppe und $R_3$ eine Hydroxylgruppe bedeuten,
welche Derivate der Formel (I/a), (I/b), (I/c) und (I/d) man mit Hilfe einer klassischen Reinigungsmethode reinigen kann und die man gegebenenfalls mit Hilfe einer klassischen Trennmethode in die Isomeren trennen kann und die man gewünschtenfalls mit einer pharmazeutisch annehmbaren Säure oder Base in ihre Säureadditionssalze umwandeln kann.

2. Verfahren nach Anspruch 1 zur Herstellung von Verbindungen, worin m den Wert 1 besitzt.

3. Verfahren nach Anspruch 1 zur Herstellung von Verbindungen, worin m den Wert 2 besitzt.

4. Verfahren nach Anspruch 1 zur Herstellung von Verbindungen, worin $R_1$ eine Aminogruppe bedeutet.

5. Verfahren nach Anspruch 1 zur Herstellung von Verbindungen, worin $R_1$ eine n-Octylgruppe und $R_3$ eine Aminogruppe bedeuten.

6. Verfahren nach Anspruch 1 zur Herstellung von Verbindungen der Formel (VI), worin $R_1$ eine höhermolekulare Alkoxygruppe darstellt, die für die Herstellung von Verbindungen der Formel (I) geeignet sind.

**Patentansprüche für folgenden Vertragsstaat : ES**

1. Verfahren zur Herstellung von Verbindungen der allgemeinen Formel (I):

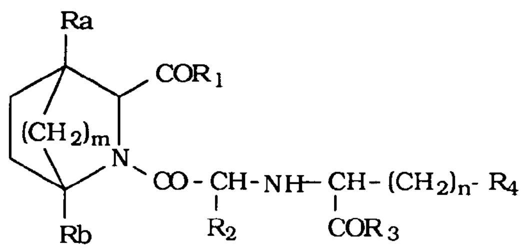

(I)

in der:
- $R_1$ eine geradkettige oder verzweigte niedrigmolekulare oder höhermolekulare Alkoxygruppe oder eine Aminogruppe, die gegebenenfalls durch eine oder zwei geradkettige oder verzweigte niedrigmolekulare Alkylgruppen substituiert ist,
- $R_2$ eine geradkettige oder verzweigte niedrigmolekulare Alkylgruppe, die gegebenenfalls durch eine Aminogruppe substituiert ist,
- $R_3$ eine Aminogruppe, eine geradkettige oder verzweigte niedrigmolekulare oder höhermolekulare Alkoxygruppe, eine Hydroxylgruppe, mit der Maßgabe, daß in $R_1$ oder $R_3$ mindestens eine Aminogruppe vorhanden ist,
- $R_4$ ein Wasserstoffatom oder eine Arylgruppe,
- m 1 oder 2,
- n zwischen 1 und 6,
- Ra und Rb, die gleichartig oder verschieden sein können, wenn m = 1, ein Wasserstoffatom, wenn m = 2, eine geradkettige oder verzweigte niedrigmolekulare Alkylgruppe oder ein Wasserstoffatom bedeuten,
  wobei der Begriff niedrigmolekular bedeutet, daß die in dieser Weise bezeichneten Gruppen 1 bis 6 Kohlenstoffatome aufweisen,
  wobei der Begriff höhermolekular bedeutet, daß die in dieser Weise bezeichneten Gruppen 7 bis 12 Kohlenstoffatome aufweisen,

sowie von deren Enantiomeren, Diastereoisomeren und Epimeren sowie von deren Additionssalzen mit einer pharmazeutisch annehmbaren Säure oder Base, **dadurch gekennzeichnet**. daß man die Verbindung der Formel (II):

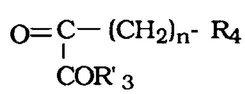

(II)

in der $R_4$ die bezüglich der Formel (I) angegebenen Bedeutungen besitzt und $R'_3$ eine Aminogruppe oder eine geradkettige oder verzweigte niedrigmolekulare oder höhermolekulare Alkoxygruppe bedeutet, in Gegenwart eines gemischten Alkalimetallhydrids, wie Natriumcyanoborhydrid, mit einer Aminosäure der Formel (III), deren Säuregruppe geschützt ist:

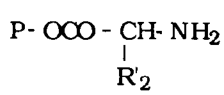

(III)

in der P eine Alkylgruppe, wie eine tert.-Butylgruppe, und $R'_2$ eine geradkettige oder verzweigte niedrigmolekulare Alkylgruppe, die gegebenenfalls durch eine Aminosäure substituiert ist, die ihrerseits durch eine Schutzgruppe, wie die Benzyloxycarbonylgruppe, geschützt ist, einer reduzierenden Aminierung unterwirft,
so daß man ein Derivat der Formel (IV) erhält:

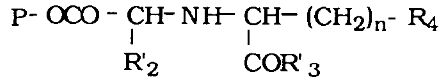

(IV)

in der $R'_2$, $R'_3$, $R_4$, P und n die oben angegebenen Bedeutungen besitzen. deren Isomere man gegebenenfalls mit Hilfe einer klassischen Trennmethode trennt und von dem man die Schutzgruppen in einem sauren Medium abspaltet, so daß man ein Derivat der Formel (V) erhält:

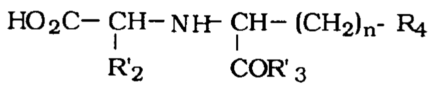

(V)

in der $R'_2$, $R'_3$, $R_4$ und n die oben angegebenen Bedeutungen besitzen, welches dann mit einem Derivat der Formel (VI):

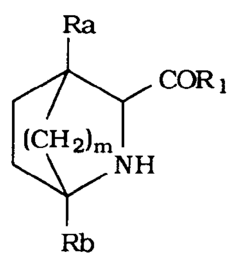

(VI)

in der Ra, Rb, m und $R_1$ die bezüglich der Formel (I) angegebenen Bedeutungen besitzen, kuppelt,
so daß man ein Derivat der Formel (I/a) erhält, einem Sonderfall der Derivate der Formel (I):

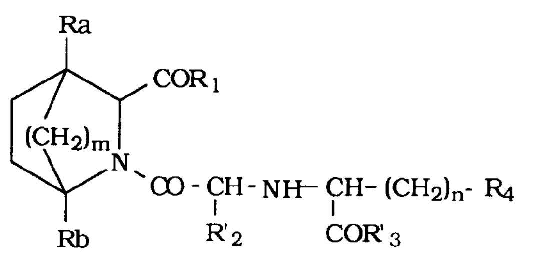

(I/a)

worin Ra, Rb, $R_1$, $R'_2$, $R'_3$, $R_4$, m und n die oben angegebenen Bedeutungen besitzen,
welches man gewünschtenfalls, wenn $R'_2$ eine geradkettige oder verzweigte niedrigmolekulare Alkylgruppe und $R'_3$ eine geradkettige oder verzweigte niedrigmolekulare oder höhermolekulare Alkoxygruppe bedeuten, der Einwirkung einer Base oder einer Säure unterwirft, so daß man ein Derivat der Formel (I/b) erhält, einem Sonderfall der Derivate der Formel (I):

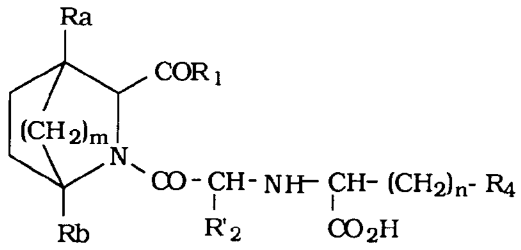

(I/b)

in der Ra, Rb, $R_1$, $R_4$, m und n die bezüglich der Formel (I) angegebenen Bedeutungen besitzen, $R'_2$ eine geradkettige oder verzweigte niedrigmolekulare Alkylgruppe und $R_3$ eine Hydroxylgruppe bedeuten, oder, wenn $R'_2$ eine durch eine geschützte Aminogruppe substituierte Alkylgruppe und $R'_3$ eine geradkettige oder verzweigte niedrigmolekulare oder höhermolekulare Alkoxygruppe bedeuten, einer Hydrierung in Gegenwart eines Katalysators, wie Palladium-auf-Kohlenstoff, unterwirft, so daß man ein Derivat der Formel (I/c) erhält, einem Sonderfall der Derivate der Formel (I):

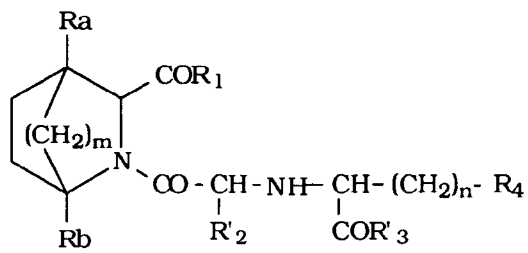

(I/c)

worin Ra, Rb, $R_1$, $R_4$, n und m die bezüglich der Formel (I) angegebenen Bedeutungen besitzen, $R'_2$ eine durch eine Aminogruppe substituierte Alkylgruppe und $R'_3$ eine geradkettige oder verzweigte niedrigmolekulare oder höhermolekulare Alkoxygruppe bedeuten,
welches Derivat (I/c) gewünschtenfalls der Einwirkung einer Base unterworfen werden kann, so daß man ein Derivat der Formel (I/d) erhält, einem Sonderfall der Derivate der Formel (I):

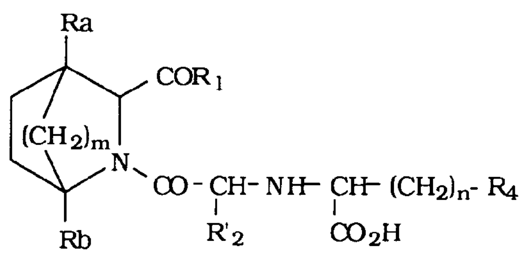

(I/d)

in der Ra, Rb, $R_1$, $R_4$, n und m die bezüglich der Formel (I) angegebenen Bedeutungen besitzen, $R'_2$ eine durch eine Aminogruppe substituierte Alkylgruppe und $R_3$ eine Hydroxylgruppe bedeuten,
welche Derivate der Formel (I/a), (I/b), (I/c) und (I/d) man mit Hilfe einer klassischen Reinigungsmethode reinigen kann und die man gegebenenfalls mit Hilfe einer klassischen Trennmethode in die Isomeren trennen kann und die man gewünschtenfalls mit einer pharmazeutisch annehmbaren Säure oder Base in ihre Säureadditionssalze umwandeln kann.

2. Verfahren nach Anspruch 1 zur Herstellung von Verbindungen, worin m den Wert 1 besitzt.

3. Verfahren nach Anspruch 1 zur Herstellung von Verbindungen, worin m den Wert 2 besitzt.

4. Verfahren nach Anspruch 1 zur Herstellung von Verbindungen, worin $R_1$ eine Aminogruppe bedeutet.

5. Verfahren nach Anspruch 1 zur Herstellung von Verbindungen, worin $R_1$ eine n-Octylgruppe und $R_3$ eine Aminogruppe bedeuten.

6. Verfahren nach Anspruch 1 zur Herstellung von Verbindungen der Formel (VI), worin $R_1$ eine höhermolekulare Alkoxygruppe darstellt, die für die Herstellung von Verbindungen der Formel (I) geeignet sind.

**Claims for the following Contracting States : AT, BE, CH, DE, DK, FR, GB, IT, LI, LU, NL, SE**

1. Compounds of the general formula (I) :

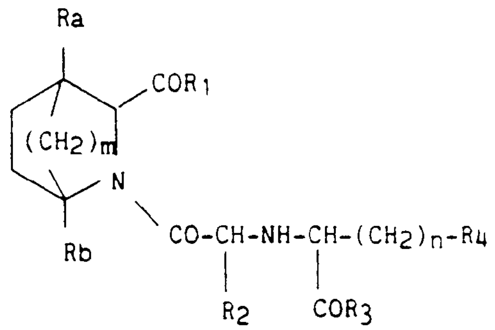

(I)

wherein :
- $R_1$ represents a straight-chain or branched lower or higher alkoxy group, or an amino group optionally substituted by one or two straight-chain or branched lower alkyl groups,
- $R_2$ represents a straight-chain or branched lower alkyl group optionally substituted by an amino group,
- $R_3$ represents an amino group, a straight-chain or branched lower or higher alkoxy group or a hydroxy group, with the proviso that at least one amino group is present in $R_1$ or $R_3$ ,
- $R_4$ represents a hydrogen atom or an aryl group,
- m is 1 or 2,
- n is from 1 to 6,
- Ra and Rb, which are the same or different, represent a hydrogen atom when m = 1 and a straight-chain or branched lower alkyl group or a hydrogen atom when m = 2,
  the term "lower" denoting that groups so qualified contain from 1 to 6 carbon atoms,
  the term "higher" denoting that groups so qualified contain from 7 to 12 carbon atoms,

their enantiomers, diastereoisomers and epimers, and also their addition salts with a pharmaceutically acceptable acid or base.

2. Compounds according to claim 1 wherein m is 1.

3. Compounds according to claim 1 wherein m is 2.

4. Compounds according to claim 1 wherein $R_1$ represents an amino group.

5. Compounds according to claim 1 wherein $R_1$ represents an n-octyl group and $R_3$ represents an amino group.

6. Process for the preparation of compounds of formula (I), characterised in that a compound of formula (II) :

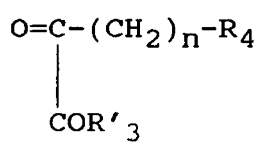

(II)

wherein $R_4$ is as defined for formula (I) and $R'_3$ represents an amino group or a straight-chain or branched lower or higher alkoxy group, is subjected to reductive amination in the presence of an alkali metal mixed hydride, such as sodium cyanoborohydride, with an amino acid of formula (III) of which the acid function is protected :

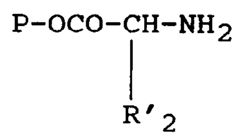

(III)

wherein P is an alkyl group such as tert-butyl and $R'_2$ is a straight-chain or branched lower alkyl group optionally substituted by an amino group which is itself protected by a protecting group such as benzyloxycarbonyl,
to obtain a compound of formula (IV) :

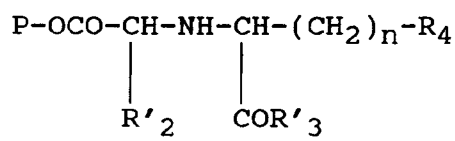

(IV)

wherein $R'_2$ , $R'_3$, $R_4$, P and n are as defined hereinbefore, of which the isomers are optionally separated by a conventional method of separation, and which is deprotected in acid medium to obtain a compound of formula (V) :

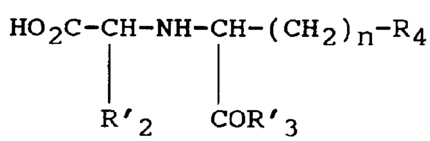

(V)

wherein $R'_2$, $R'_3$, $R_4$ and n are as defined hereinbefore,
which is then coupled with a compound of formula (VI) :

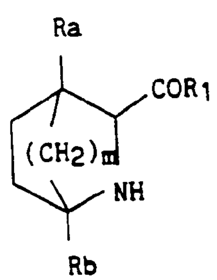

(VI)

wherein Ra, Rb, m and $R_1$ are as defined for formula (I),
to yield a compound of formula (I/a), a particular case of compounds of formula (I) :

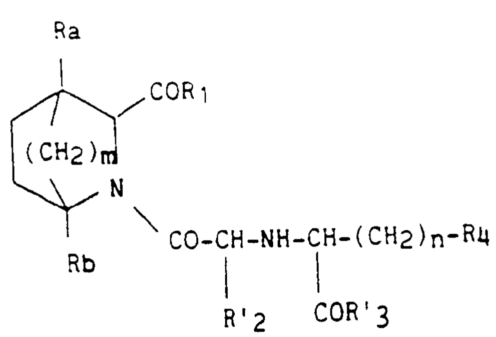

(I/a)

wherein Ra, Rb, $R_1$, $R'_2$, $R'_3$, $R_4$, m and n are as defined hereinbefore,
which, if desired, when $R'_2$ represents a straight-chain or branched lower alkyl group and $R'_3$ represents a straight-chain or branched lower or higher alkoxy group, may be subjected to the action of a base or an

acid to yield a compound of formula (I/b), a particular case of compounds of formula (I) :

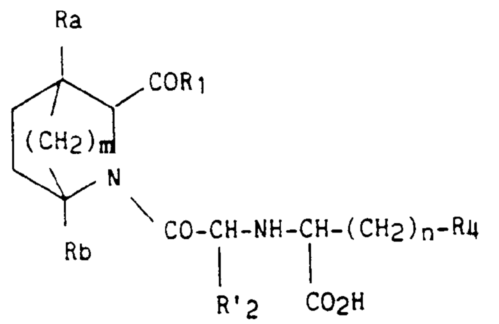

(I/b)

wherein Ra, Rb, $R_1$, $R_4$, m and n are as defined for formula (I), $R'_2$ represents a straight-chain or branched lower alkyl group and $R_3$ represents a hydroxy group,
or, when $R'_2$ is an alkyl group substituted by a protected amino group and $R'_3$ is a straight-chain or branched lower or higher alkoxy group, may be subjected to hydrogenation in the presence of a catalyst, such as palladium-on-carbon, to yield a compound of formula (I/c), a particular case of compounds of formula (I) :

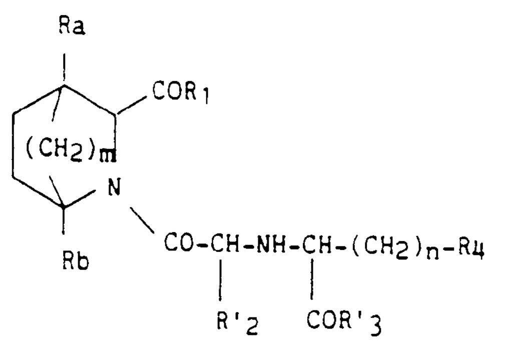

(I/c)

wherein Ra, Rb, $R_1$, $R_4$, n and m are as defined for formula (I), $R'_2$ represents an alkyl group substituted by an amino group and $R'_3$ represents a straight-chain or branched lower or higher alkoxy group,
which compound (I/c) may, if desired, be subjected to the action of a base to yield a compound of formula (I/d), a particular case of compounds of formula (I) :

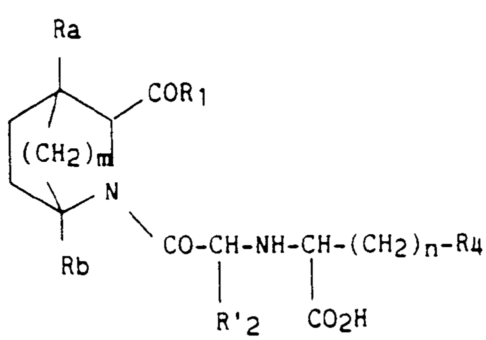

(I/d)

wherein Ra, Rb, $R_1$, $R_4$, n and m are as defined for formula (I), $R'_2$ represents an alkyl group substituted by an amino group and $R_3$ represents a hydroxy group,
which compounds of formula (I/a), (I/b), (I/c) and (I/d) are purified by a conventional method of purification, are optionally separated into their isomers by a conventional method of separation, and, if desired, are converted into their addition salts with a pharmaceutically acceptable acid or base.

7. Compounds of formula (VI) wherein $R_1$ represents a higher alkoxy group, for use in the preparation of compounds of formula (I).

**8.** Pharmaceutical compositions containing as active ingredient at least one compound according to any one of claims 1 to 5, alone or in combination with one or more pharmaceutically acceptable, inert, non-toxic excipients or carriers.

**9.** Pharmaceutical compositions according to claim 8 containing at least one active ingredient according to any one of claims 1 to 5, for use in the treatment of arterial hypertension, and neurobehavioural disorders associated with ageing and with senile or presenile degenerative dementias, such as Alzheimer's disease, Pick's disease, dementia multi-infarctus and Binswanger's disease.

**Claims for the following Contracting State : GR**

**1.** Process for the preparation of compounds of the general formula (I) :

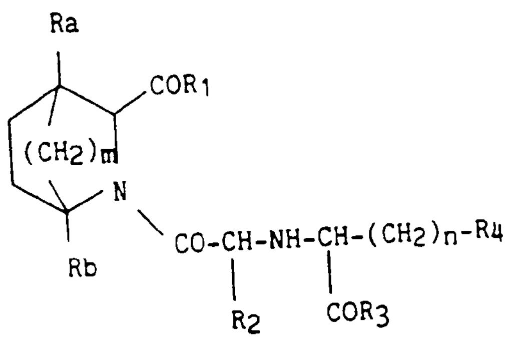

(I)

wherein :

- $R_1$ represents a straight-chain or branched lower or higher alkoxy group, or an amino group optionally substituted by one or two straight-chain or branched lower alkyl groups,
- $R_2$ represents a straight-chain or branched lower alkyl group optionally substituted by an amino group,
- $R_3$ represents an amino group, a straight-chain or branched lower or higher alkoxy group or a hydroxy group, with the proviso that at least one amino group is present in $R_1$ or $R_3$,
- $R_4$ represents a hydrogen atom or an aryl group,
- m is 1 or 2,
- n is from 1 to 6,
- Ra and Rb, which are the same or different, represent a hydrogen atom when m = 1 and a straight-chain or branched lower alkyl group or a hydrogen atom when m = 2,
  the term "lower" denoting that groups so qualified contain from 1 to 6 carbon atoms,
  the term "higher" denoting that groups so qualified contain from 7 to 12 carbon atoms,

their enantiomers, diastereoisomers and epimers, and also their addition salts with a pharmaceutically acceptable acid or base, characterised in that a compound of formula (II) :

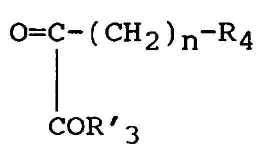

(II)

wherein $R_4$ is as defined for formula (I) and $R'_3$ represents an amino group or a straight-chain or branched lower or higher alkoxy group, is subjected to reductive amination in the presence of an alkali metal mixed hydride, such as sodium cyanoborohydride, with an amino acid of formula (III) of which the acid function is protected :

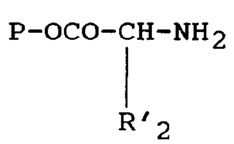

(III)

wherein P is an alkyl group such as tert-butyl and $R'_2$ is a straight-chain or branched lower alkyl group optionally substituted by an amino group which is itself protected by a protecting group such as benzyloxycarbonyl,
to obtain a compound of formula (IV) :

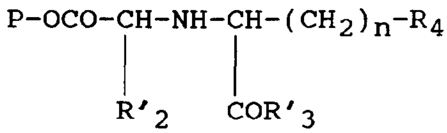

(IV)

wherein $R'_2$, $R'_3$, $R_4$, P and n are as defined hereinbefore, of which the isomers are optionally separated by a conventional method of separation, and which is deprotected in acid medium to obtain a compound of formula (V) :

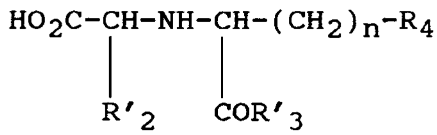

(V)

wherein $R'_2$, $R'_3$, $R_4$ and n are as defined hereinbefore,
which is then coupled with a compound of formula (VI) :

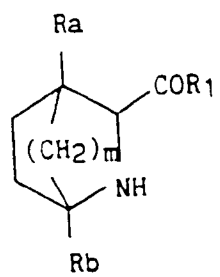

(VI)

wherein Ra, Rb, m and $R_1$ are as defined for formula (I),
to yield a compound of formula (I/a), a particular case of compounds of formula (I) :

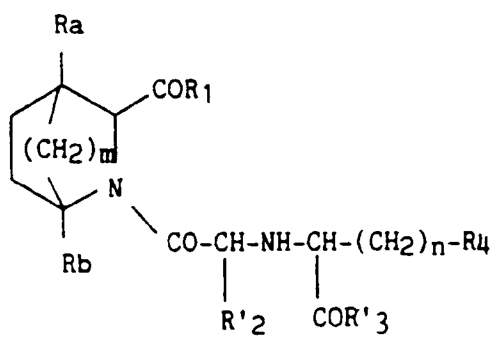

(I/a)

wherein Ra, Rb, $R_1$, $R'_2$, $R'_3$, $R_4$, m and n are as defined hereinbefore,
which, if desired, when $R'_2$ represents a straight-chain or branched lower alkyl group and $R'_3$ represents a straight-chain or branched lower or higher alkoxy group, may be subjected to the action of a base or an acid to yield a compound of formula (I/b), a particular case of compounds of formula (I) :

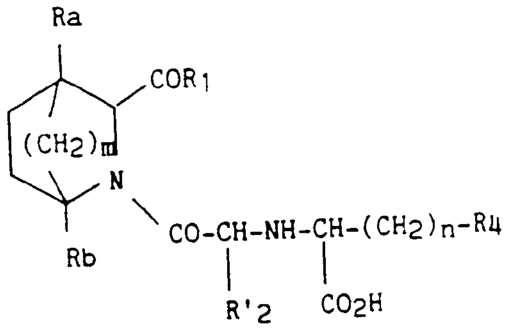

(I/b)

wherein Ra, Rb, $R_1$, $R_4$, m and n are as defined for formula (I), $R'_2$ represents a straight-chain or branched lower alkyl group and $R_3$ represents a hydroxy group,
or, when $R'_2$ is an alkyl group substituted by a protected amino group and $R'_3$ is a straight-chain or branched lower or higher alkoxy group, may be subjected to hydrogenation in the presence of a catalyst, such as palladium-on-carbon, to yield a compound of formula (I/c), a particular case of compounds of formula (I) :

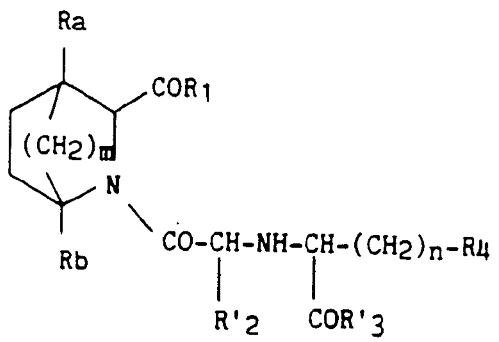

(I/c)

wherein Ra, Rb, $R_1$, $R_4$, n and m are as defined for formula (I), $R'_2$ represents an alkyl group substituted by an amino group and $R'_3$ represents a straight-chain or branched lower or higher alkoxy group,
which compound (I/c) may, if desired, be subjected to the action of a base to yield a compound of formula (I/d), a particular case of compounds of formula (I) :

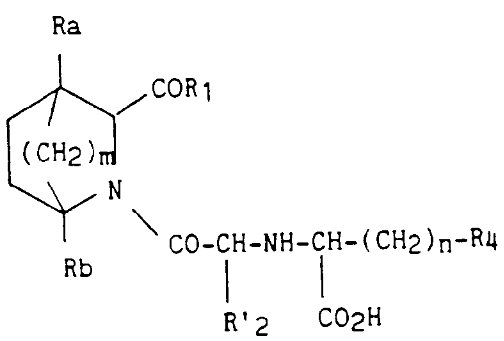

(I/d)

wherein Ra, Rb, $R_1$, $R_4$, n and m are as defined for formula (I), $R'_2$ represents an alkyl group substituted by an amino group and $R_3$ represents a hydroxy group,
which compounds of formula (I/a), (I/b), (I/c) and (I/d) are purified by a conventional method of purification, are optionally separated into their isomers by a conventional method of separation, and, if desired, are converted into their addition salts with a pharmaceutically acceptable acid or base.

2. Process according to claim 1 for the preparation of compounds wherein m is 1.

3. Process according to claim 1 for the preparation of compounds wherein m is 2.

4. Process according to claim 1 for the preparation of compounds wherein $R_1$ represents an amino group.

5. Process according to claim 1 for the preparation of compounds wherein $R_1$ represents an n-octyl group and $R_3$ represents an amino group.

6. Process according to claim 1 for the preparation of compounds of formula (VI) wherein $R_1$ represents a higher alkoxy group for use in the preparation of compounds of formula (I).

**Claims for the following Contracting State : ES**

1. Process for the preparation of compounds of the general formula (I) :

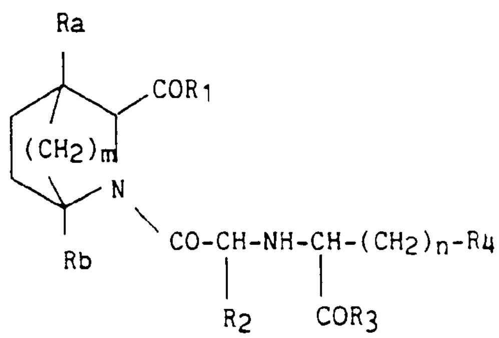

(I)

wherein :
- $R_1$ represents a straight-chain or branched lower or higher alkoxy group, or an amino group optionally substituted by one or two straight-chain or branched lower alkyl groups,
- $R_2$ represents a straight-chain or branched lower alkyl group optionally substituted by an amino group,
- $R_3$ represents an amino group, a straight-chain or branched lower or higher alkoxy group or a hydroxy group,
  with the proviso that at least one amino group is present in $R_1$ or $R_3$,
- $R_4$ represents a hydrogen atom or an aryl group,
- m is 1 or 2,
- n is from 1 to 6,
- Ra and Rb, which are the same or different, represent a hydrogen atom when m = 1 and a straight-chain or branched lower alkyl group or a hydrogen atom when m = 2,
  the term "lower" denoting that groups so qualified contain from 1 to 6 carbon atoms,
  the term "higher" denoting that groups so qualified contain from 7 to 12 carbon atoms,

their enantiomers, diastereoisomers and epimers, and also their addition salts with a pharmaceutically acceptable acid or base, characterised in that a compound of formula (II) :

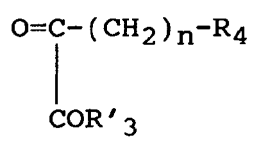

(II)

wherein $R_4$ is as defined for formula (I) and $R'_3$ represents an amino group or a straight-chain or branched lower or higher alkoxy group, is subjected to reductive amination in the presence of an alkali metal mixed hydride, such as sodium cyanoborohydride, with an amino acid of formula (III) of which the acid function is protected :

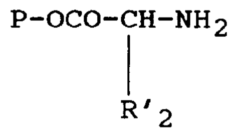

(III)

wherein P is an alkyl group such as tert-butyl and $R'_2$ is a straight-chain or branched lower alkyl group optionally substituted by an amino group which is itself protected by a protecting group such as benzyloxycarbonyl,
to obtain a compound of formula (IV) :

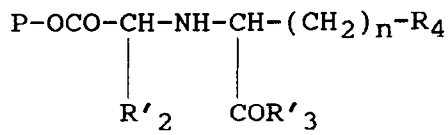

(IV)

wherein $R'_2$, $R'_3$, $R_4$, P and n are as defined hereinbefore, of which the isomers are optionally separated by a conventional method of separation, and which is deprotected in acid medium to obtain a compound of formula (V) :

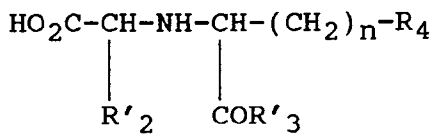

(V)

wherein $R'_2$, $R'_3$, $R_4$ and n are as defined hereinbefore,
which is then coupled with a compound of formula (VI) :

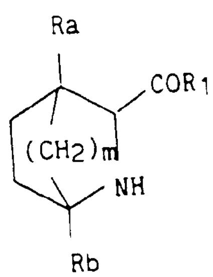

(VI)

wherein Ra, Rb, m and $R_1$ are as defined for formula (I),
to yield a compound of formula (I/a), a particular case of compounds of formula (I) :

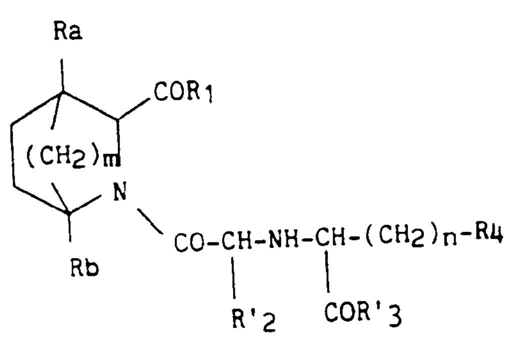

(I/a)

wherein Ra, Rb, $R_1$, $R'_2$, $R'_3$, $R_4$, m and n are as defined hereinbefore, which, if desired, when $R'_2$ represents a straight-chain or branched lower alkyl group and $R'_3$ represents a straight-chain or branched lower or higher alkoxy group, may be subjected to the action of a base or an acid to yield a compound of formula (I/b), a particular case of compounds of formula (I) :

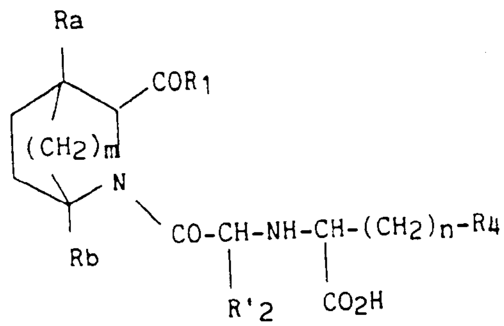

(I/b)

wherein Ra, Rb, $R_1$, $R_4$, m and n are as defined for formula (I), $R'_2$ represents a straight-chain or branched lower alkyl group and $R_3$ represents a hydroxy group, or, when $R'_2$ is an alkyl group substituted by a protected amino group and $R'_3$ is a straight-chain or branched lower or higher alkoxy group, may be subjected to hydrogenation in the presence of a catalyst, such as palladium-on-carbon, to yield a compound of formula (I/c), a particular case of compounds of formula (I) :

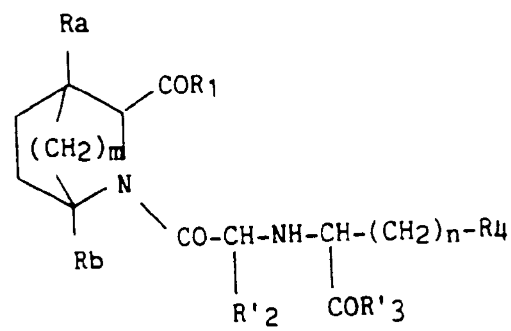

(I/c)

wherein Ra, Rb, $R_1$, $R_4$, n and m are as defined for formula (I), $R'_2$ represents an alkyl group substituted by an amino group and $R'_3$ represents a straight-chain or branched lower or higher alkoxy group, which compound (I/c) may, if desired, be subjected to the action of a base to yield a compound of formula (I/d), a particular case of compounds of formula (I) :

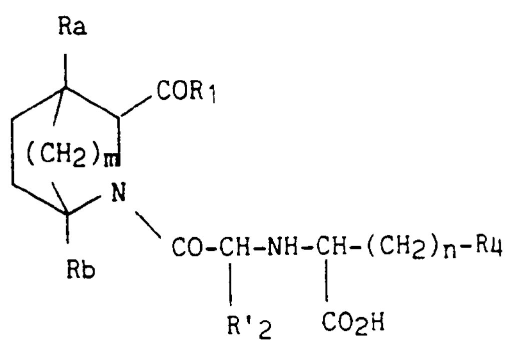

(I/d)

wherein Ra, Rb, $R_1$, $R_4$, n and m are as defined for formula (I), $R'_2$ represents an alkyl group substituted by an amino group and $R_3$ represents a hydroxy group, which compounds of formula (I/a), (I/b), (I/c) and (I/d) are purified by a conventional method of purification, are optionally separated into their isomers by a conventional method of separation, and, if desired, are converted into their addition salts with a pharmaceutically acceptable acid or base.

2. Process according to claim 1 for the preparation of compounds wherein m is 1.

3. Process according to claim 1 for the preparation of compounds wherein m is 2.

4. Process according to claim 1 for the preparation of compounds wherein $R_1$ represents an amino group.

5. Process according to claim 1 for the preparation of compounds wherein $R_1$ represents an n-octyl group and $R_3$ represents an amino group.

6. Process according to claim 1 for the preparation of compounds of formula (VI) wherein $R_1$ represents a higher alkoxy group for use in the preparation of compounds of formula (I).